# EUROPÄISCHE PATENTSCHRIFT

(11) **EP 0 450 742 B1**
(45) Veröffentlichungstag und Bekanntmachung des Hinweises auf die Patenterteilung: **06.07.1994**
(21) Anmeldenummer: 91250089.9
(22) Anmeldetag: 05.04.1991
(51) Int. Cl.: C07C 237/04, C07C 237/12, C07C 309/15, C07C 309/51, A61K 49/00, A61K 49/02, A61K 49/04

(54) **DTPA-Monoamide, diese Verbindungen enthaltende pharmazeutische Mittel, ihre Verwendung und Verfahren zu deren Herstellung**
DTPA-Monoamids, pharmaceutical compositions containing them, their use and process for their preparation
DTPA-monoamides, compositions pharmaceutiques les contenant, leur utilisation et procédé pour leur préparation

(30) Priorität: 06.04.1990 DE 4011684
(43) Veröffentlichungstag der Anmeldung: 09.10.1991
(73) Patentinhaber: SCHERING AKTIENGESELLSCHAFT, 13342 Berlin (DE)
(72) Erfinder: Gries, Heinz, Dr., W-1000 Berlin 31 (DE); Klieger, Erich, Dr., W-1000 Berlin 30 (DE); Radüchel, Bernd, Dr., W-1000 Berlin 28 (DE); Schmitt-Willich, Heribert, Dr., W-1000 Berlin 45 (DE); Weinmann, Hanns-Joachim, Dr., W-1000 Berlin 38 (DE); Vogler, Hubert, Dr., W-1000 Berlin 12 (DE); Schuhmann-Giampieri, Gabriele, Dr., W-1000 Berlin 45 (DE); Conrad, Jürgen, Dr., W-1000 Berlin 41 (DE)

(56) Entgegenhaltungen:
- EP-A- 0 130 934
- EP-A- 0 263 059
- EP-A- 0 405 704
- WO-A-88/07521
- GB-A- 2 060 623

## Beschreibung

Die Erfindung betrifft den in den Patentansprüchen gekennzeichneten Gegenstand, das heißt neue Diethylentriaminpentaessigsäure (DTPA)-Monoamide, DTPA-monoamid-Komplexe und -Komplexsalze, diese Verbindungen enthaltende Mittel, ihre Verwendung in der NMR-Diagnostik sowie Verfahren zur Herstellung dieser Verbindungen und Mittel.

Metallkomplexe sind schon zu Beginn der 50er Jahre als Kontrastmittel für die Radiologie in Betracht gezogen worden. Die damals eingesetzten Verbindungen waren aber derart toxisch, daß eine Verwendung beim Menschen nicht in Betracht kam. Es war daher durchaus überraschend, daß sich bestimmte Komplexsalze als ausreichend verträglich erwiesen haben, so daß eine routinemäßige Anwendung am Menschen für diagnostische Zwecke in Erwägung gezogen werden konnte. Als erster Vertreter dieser Substanzklasse hat sich das in der europäischen Patentanmeldung mit der Publikationsnummer 71564 beschriebene Dimegluminsalz des Gd DTPA (Gadolinium-III-Komplex der Diethylentriaminpentaessigsäure) als Kontrastmittel für die Kernspintomographie sehr gut bewährt. Es ist unter dem Namen Magnevist^{(R)} weltweit als erstes NMR-Diagnostikum registriert worden.

Magnevist^{(R)} ist besonders gut für die Diagnose pathologischer Bereiche (z.B. Entzündungen, Tumore, Infarkte etc.) geeignet. Nach intravenöser Injektion verteilt sich die Verbindung extrazellulär und wird durch glomeruläre Sekretion über die Nieren eliminiert. Eine Passage intakter Zellmembranen und eine extrarenale Ausscheidung werden praktisch nicht beobachtet.

Besonders für Patienten mit eingeschränkter Nierenfunktion, bei denen Magnevist^{(R)} nur sehr langsam ausgeschieden wird und zum Teil nur mit Hilfe eines Dialysegerätes aus dem Organismus entfernt werden kann, wären Kontrastmittel, die eine zumindest nur teilweise extrarenale Ausscheidung aufweisen, wünschenswert.

Es besteht daher ein Bedarf an NMR-Kontrastmitteln, die ein anderes pharmakokinetisches Verhalten als Magnevist^{(R)} zeigen.

Der Erfindung liegt somit die Aufgabe zugrunde, derartige Verbindungen und Mittel zur Verfügung zu stellen sowie ein Verfahren zu ihrer Herstellung zu schaffen. Diese Aufgabe wird durch die vorliegende Erfindung erfüllt.

Es wurde gefunden, daß die erfindungsgemäßen Verbindungen überraschenderweise die gewünschte Eigenschaft zeigen: sowohl renale Ausscheidung als auch Exkretion mit den Faeces nach parenteraler Verabreichung. Überraschenderweise ist die Elimination über die Galle jedoch nicht der einzige extrarenale Ausscheidungsweg: bei NMR-Untersuchungen an Ratten wurde nach intravenöser Gabe der erfindungsgemäßen Verbindungen unerwartet auch eine Kontrastverstärkung des Magen-Darm-Traktes beobachtet, d.h. diese Verbindungen sind sowohl für die organspezifische NMR-Diagnostik des hepatobiliären Systems als auch des Magens geeignet. Die Nieren sowie implantierte Tumore werden ebenfalls kontrastiert.

Überraschenderweise werden die erfindungsgemäßen Komplexverbindungen auch nach oraler Applikation resorbiert und anschließend hepatobiliär ausgeschieden, d.h. sie sind als orale Leberkontrastmittel geeignet.

Ein derartiges Phänomen wurde bisher von keinem Kontrastmittel für die Kernspintomographie beschrieben. Die Ausscheidung (Sekretion) über den Magen hat den Vorteil, daß eine Abgrenzung abdominaler Strukturen (z.B. Pankreas) vom Gastrointestinal-Trakt mit gleichzeitiger Kontrastverstärkung pathologischer Prozesse (Tumoren, Entzündungen) ermöglicht wird. Eine Darstellung des renalen Systems, der Leber und der Gallenblase und Gallenwege sowie der Lymphknoten kann darüber hinaus auch erzielt werden. Neben der verbesserten Darstellung von Ulci und Magenkarzinomen kann auch eine Überprüfung der Magensaftsekretion mit Hilfe bildgebender Verfahren durchgeführt werden.

Durch die Verwendung der o.g. Verbindungen kann - neben der Diagnostik des hepatobiliären Systems - somit sowohl niereninsuffizienten als auch unter gastrointestinalen Erkrankungen leidenden Patienten (mindestens 10 % der Bevölkerung in den westlichen Industrieländern) geholfen werden. Die meisten dieser Patienten sowie eine große Anzahl von Patienten, bei denen der Verdacht auf eine solche Erkrankung vorliegt, müssen sich diagnostischen Untersuchungen unterziehen. Zur Zeit sind für gastrointestinale Erkrankungen vor allem zwei dafür geeigneter Methoden gebräuchlich: die Endoskopie und die Röntgenstrahl-Diagnostik mit Hilfe von Barium-Kontrastmitteln.

Diese Untersuchungen weisen verschiedene Nachteile auf: sie sind mit dem Risiko der Strahlenbelastung behaftet, trauma-verursachend, mit Unannehmlichkeiten, gelegentlich sogar mit Risiken für den Patienten verbunden und können daher psychologischen Streß hervorrufen. Sie müssen meist wiederholt durchgeführt werden, sind relativ aufwendig durchzuführen, erfordern die aktive Mitarbeit des Patienten (z.B. Einnehmen einer bestimmten Körperhaltung) und sind oft nicht anwendbar bei gebrechlichen und bei Risiko-Patienten.

Die Aufgabe, neue diagnostische Methoden zur Erkennung und Lokalisierung gastrointestinaler Erkrankungen, die diese Nachteile nicht besitzen, zur Verfügung zu stellen, wird daher durch die Verwendung der o.g. Komplexverbindungen und Mittel ebenfalls gelöst.

Auch ohne spezifische Maßnahmen erlaubt ihre Pharmakokinetik die Verbesserung der Diagnose zahlreicher Erkrankungen. Die Komplexe werden zum größten Teil unverändert und rasch wieder ausgeschieden, so daß insbesondere auch im Falle der Verwendung relativ toxischer Metallionen auch bei hoher Dosierung keine schädlichen Wirkungen beobachtet werden.

Die praktische Anwendung der neuen Komplexe wird auch durch deren günstige chemische Stabilität erleichtert.

Die erfindungsgemäßen Verbindungen werden durch die allgemeinen Formel I gekennzeichnet: 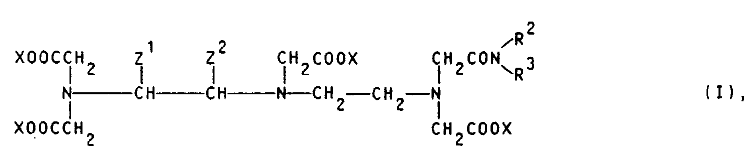
worin
- Z¹ und Z²: jeweils für ein Wasserstoffatom oder den Rest

-(CH₂)ₘ-(C₆H₄)_{q}-(O)ₖ-(CH₂)ₙ-(C₆H₄)ₗ-(O)ᵣ-R,

worin
m und n die Ziffern 0 - 20,
k, l, q und r die Ziffern 0 und 1 und
R ein Wasserstoffatom, einen gegebenenfalls OR¹-substituierten C₁-C₆-Alkylrest oder eine CH₂COOR¹-Gruppe mit R¹ in der Bedeutung eines Wasserstoffatoms, eines C₁-C₆-Alkylrestes oder einer Benzylgruppe bedeuten,
- R²: für eine durch eine gegebenenfalls mit einem C₁-C₆-Alkyl- oder Benzylrest veresterte Carboxyl- oder Sulfogruppe substituierte gesättigte, ungesättigte, gerad- oder verzweigtkettige oder cyclische Alkylgruppe mit bis zu 20 C-Atomen, Aryl- oder Aralkylgruppe,
- R³: für ein Wasserstoffatom oder für eine gegebenenfalls durch eine gegebenenfalls mit einem C₁-C₆-Alkyl- oder Benzylrest veresterte Carboxyl- oder Sulfogruppe substituierte gesättigte, ungesättigte, gerad- oder verzweigtkettige oder cyclische Alkylgruppe mit bis zu 20 C-Atomen, Aryl- oder Aralkylgruppe,
- X: für ein Wasserstoffatom und/oder ein Metallionenäquivalent eines Elements der Ordnungszahlen 21-29, 31, 32, 37-40, 42-44, 49 oder 57-83 stehen, mit der Maßgabe, daß mindestens einer der Substituenten Z¹ und Z² für ein Wasserstoffatom steht, daß - wenn n und l jeweils für die Ziffer 0 stehen - k und r nicht gleichzeitig jeweils die Ziffer 1 bedeuten und daß gewünschtenfalls der Rest der Säuregruppen als Ester oder Amid vorliegt, sowie deren Salze mit anorganischen und/oder organischen Basen, Aminosäuren oder Aminosäureamide.

Verbindungen der allgemeinen Formel I mit X in der Bedeutung von Wasserstoff werden als Komplexbildner und mit mindestens zwei der Substituenten X in der Bedeutung eines Metallionenäquivalents als Metallkomplexe bezeichnet.

Das Element der oben genannten Ordnungszahl, welches das Zentralion des physiologisch verträglichen Komplexsalzes bildet, kann für den angestrebten Verwendungszweck des erfindungsgemäßen diagnostischen Mittels selbstverständlich auch radioaktiv sein.

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21-29 42, 44 und 58-70. Geeignete Ionen sind beispielsweise das Chrom(III)-, Mangan(II)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)- und Eisen(III)-ion.

Für die Verwendung der erfindungsgemäßen Mittel in der Nuklearmedizin muß das Zentralion radioaktiv sein. Geeignet sind zum Beispiel Radioisotope der Elemente Kupfer, Kobalt, Gallium, Germanium, Yttrium, Strontium, Technetium, Indium, Ytterbium, Gadolinium, Samarium und Iridium.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 42, 44, 57-83 enthalten, geeignet sind; dies sind beispielsweise das Lanthan(III)-ion und die oben genannten Ionen der Lanthanidenreihe.

Die für m und n stehenden Ziffern sind bevorzugt 0 bis 5.

Als Alkylsubstituenten R, R¹ und den in R² gegebenenfalls enthaltenden Alkylsubstituenten kommen geradkettige oder verzweigte Kohlenwasserstoffe mit bis zu 6, bevorzugt bis zu 4, Kohlenstoffatomen, die im Falle von R gegebenenfalls durch eine oder mehrere, bevorzugt 1 bis 3, Hydroxy- oder C₁-C₆-, bevorzugt C₁-C₄-Alkoxygruppen substituiert sind, infrage.

Als gegebenenfalls substituierte Alkylgruppen seien beispielsweise die Methyl-, Hydroxymethyl-, Ethyl-, 2-Hydroxyethyl-, 2-Hydroxy-1-(hydroxymethyl)-ethyl-, 1-(Hydroxymethyl)-ethyl-, Propyl-, Isopropyl-, 2- und 3-Hydroxypropyl, 2,3-Dihydroxypropyl-, n-, sek.- und tert.-Butyl-, 2-, 3-, und 4-Hydroxybutyl-, 2- und 3-Hydroxy-isobutyl, Pentyl-, 2-,3- und 4-Hydroxy-2-methylbutyl-, 2,3,4-Trihydroxybutyl-, 1,2,4-Trihydroxybutyl-, Cyclopentyl-, Cyclohexyl-, 2,3,4,5,6-Pentahydroxyhexylgruppe sowie - im Falle der Hydroxyalkylgruppen - deren C₁-C₆-, bevorzugt C₁-C₄-Alkylderivate, genannt.

Als bevorzugte Aryl- und Aralkylgruppen R² und R³ seien die Phenyl-, Benzyl- und Isopropylphenylreste genannt.

Bevorzugte Substituenten Z¹ bzw. Z² sind der -CH₂-C₆H₄-OH, -CH₂-C₆H₄-OCH₃, -CH₂-C₆H₅, -CH₂-C₆H₄-O-CH₂-C₆H₄-OCH₃, -CH₂-O-CH₂-C₆H₅, -CH₂-C₆H₄-O-CH₂-COOH, -CH₂-C₆H₄-OC₂H₅, -CH₂-C₆H₄-OC₄H₉, -CH₂-C₆H₄-O-CH₂-C₆H₅ Rest.

Als durch eine gegebenenfalls mit einem C₁-C₆-, bevorzugt C₁-C₄-, Alkyl- oder Benzylrest veresterte Carboxyl- oder Sulfogruppe substituierte (R²) bzw. durch eine dieser gegebenenfalls veresterten Säuregruppen gegebenenfalls (R³) substituierte Reste R² und R³ kommen gesättigte, ungesättigte, gerad-, verzweigtkettige oder cyclische Alkylgruppen mit bis zu 20 C-Atomen, vorzugsweise mit 5 bis 18 C-Atomen, Aryl- und Aralkylgruppen infrage.

Beispielsweise genannt seien der Methyl-, Ethyl-, Propyl-, Isopropyl-, Butyl-, Pentyl-, Hexyl-, Phenyl-, Benzyl-, 4-Carboxy-phenylen-, 1-Cyclohexyl-1-carbonsäure-, 1-Cyclopentyl-1-carbonsäure-, 2-Carboxy-phenylen-, 11-Carboxy-1-undecyl-, 10-Carboxy-1-decyl-, 7-Carboxy-1-heptyl-, 6-Carboxy-1-hexyl-, 5-Carboxy-1-pentyl-, 4-Carboxy-benzyl-, 4-Carboxy-1-cyclohexylmethyl-, 4-Carboxymethyl-phenylen-, 4-(1,1-Dimethylcarboxymethylen)-phenylen-, 4-Sulfo-phenylen-, 10-Sulfo-1-decyl-, 8-Sulfo-1-octyl-, 17-Carboxy-7-heptadecyl-, 7-Carboxy-2-heptyl-, 1-Carboxy-4-phenyl-2-propyl-, 2-Carboxyethyl-, 4-Carboxy-4-but-1-enyl-Rest.

Die restlichen aciden Wasserstoffatome, das heißt diejenigen, die nicht durch das Zentralion substituiert worden sind, können gegebenenfalls ganz oder teilweise durch Kationen anorganischer und/oder organischer Basen oder Aminosäuren ersetzt sein. Die entsprechenden Säuregruppen können auch ganz oder teilweise in Ester oder Amide überführt sein.

Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Geeignete Ester sind vorzugsweise diejenigen mit einem C₁-C₆-Alkylrest; genannt seien beispielsweise der Methyl-, Ethyl- und tertiär-Butyl-, Benzyl-und 4-Methoxybenzylrest.

Sollen die Carbonsäuregruppen zumindest teilweise als Amide vorliegen, so kommen als Reste gesättigte, ungesättigte, gerad- oder verzweigtkettige oder cyclische Kohlenwasserstoffe mit bis zu 5 C-Atomen infrage, die gegebenenfalls durch 1 bis 3 Hydroxy- oder C₁-C₄-Alkoxy-Gruppen substituiert sind. Beispielsweise genannt seien die Methyl-, Ethyl-, 2-Hydroxyethyl-, 2-Hydroxy-1-(hydroxymethyl)-ethyl-, 1-(Hydroxymethyl)-ethyl-, Propyl-, Isopropenyl-, 2-Hydroxypropyl-, 3-Hydroxypropyl-, 2,3-Dihydroxypropyl-, Butyl-, Isobutyl-, Isobutenyl-, 2-Hydroxybutyl-, 3-Hydroxybutyl-, 4-Hydroxybutyl-, 2-, 3- und 4-Hydroxy-2-methylbutyl-, 2- und 3-Hydroxyisobutyl-, 2,3,4-Trihydroxybutyl-, 1,2,4-Trihydroxybutyl-, Pentyl-, Cyclopentyl- und 2-Methoxyethylgruppe. Der Amidrest kann auch ein unter Einschluß des Amid-Stickstoffs gebildeter heterocyclischer 5- oder 6-Ring sein. Beispielhaft genannt seien: der Pyrrolidinyl-, Piperidyl-, Pyrazolidinyl-, Pyrrolinyl-, Pyrazolinyl-, Piperazinyl-, Morpholinyl-, Imidazolidinyl-, Oxazolidinyl-, Thiazolidinyl-Ring.

Die erfindungsgemäßen Verbindungen weisen die eingangs geschilderten gewünschten Eigenschaften auf. Darüber hinaus ist überraschenderweise die Verträglichkeit der erfindungsgemäßen Monoamide im Vergleich zu den entsprechenden Bisamiden erhöht. So liegt beispielsweise der LD₅₀-Wert (Ratte) der Verbindung des Beispiels 1b) um den Faktor 3 höher als der entsprechende Wert des vorbekannten (DE-OS 34 01 052.1, Beispiel 2) Bisamids. Zu erwarten aufgrund des Stands der Technik wäre dagegen eine Verschlechterung des LD₅₀-Werts gewesen: so sinkt beispielsweise der LD₅₀ (Maus)- bzw. LD₅₀(Ratte)-Wert des Bisamids Gadolinium-Komplex der N⁶-Carboxymethyl-N³,N⁹-bis(2,3-dihydroxypropyl-N-methyl-carbamoylmethyl)-3,6,9-triazaundecandisäure [Europäische Patentschrift Publikations-Nr. 0 130 934, Beispiel 6a)] um 50 bzw. 30 % im Falle des entsprechenden Monoamids (Formel I, Z¹=Z²=H, R² = -CH₂CH(OH)CH₂OH, R³ = CH₃).

Die Einführung von Amidgruppen zur Herstellung der Komplexbildner, d.h. von Verbindungen der allgemeinen Formel I mit X in der Bedeutung von Wasserstoff, erfolgt durch partielle Umwandlung von aktivierten Carboxylgruppen in Amidgruppen der - entsprechend dem gewünschten Endprodukt - jeweils geeigneten Pentacarbonsäuren. Für diesen Prozeß kommen alle dem Fachmann bekannten Synthesemöglichkeiten in Betracht.

Ein Beispiel hierfür ist die Umsetzung der Anhydride oder Ester der allgemeinen Formeln II und III 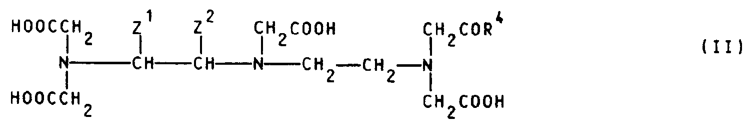 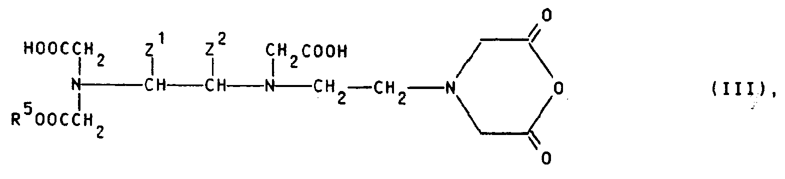
worin
- Z¹ und Z²: die oben angegebenen Bedeutungen haben,
- R⁴: für den Rest einer aktivierten Carbonylgruppe oder für OR⁵ steht und
- R⁵: ein Wasserstoffatom oder einen C₁-C₆-Alkylrest darstellt, mit Aminosäuren der allgemeinen Formel IV 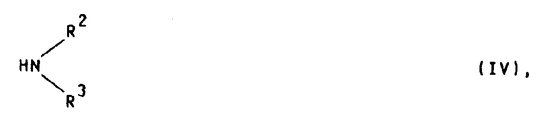 worin
- R² und R³: die oben genannten Bedeutungen haben.

Als Beispiele für eine aktivierte Carbonylgruppe seien gemischtes Anhydrid (mit z.B. Chlorameisensäureester) und das Additionsprodukt an Carbodiimide, z.B. Dicyclohexylcarabodiimid (DCC) angeführt.

Als geeignete Aminosäuren seien beispielsweise genannt:
4-Amino-benzoesäure, 1-Aminocyclohexan-1-carbonsäure, 1-Aminocyclopentan-1-carbonsäure, 2-Aminobenzoesäure, 12-Aminododecansäure, 11-Aminoundecansäure, 8-Aminooctansäure, 7-Aminoheptansäure, 6-Aminohexansäure, 4-Aminomethyl-benzoesäure, trans-4-(Aminomethyl)-cyclohexancarbonsäure, p-Aminophenylessigsäure, α,α-Dimethyl-p-aminophenylessigsäure, Sulfanilsäure, 10-Amino-decan-1-sulfonsäure, 8-Amino-octan-1-sulfonsäure, 12-Amino-octadecansäure, 2-Aminooctansäure, 2-Amino-4-phenylbuttersäure, Iminodiethansäure, N-Methylaminobenzol-4-sulfonsäure, 12-Methylamino-dodecansäure, 2-Amino-4-pentensäure.

Die Verseifung gegebenenfalls noch vorhandener Estergruppen erfolgt nach den dem Fachmann bekannten Verfahren, beispielsweise durch alkalische Hydrolyse.

Die Herstellung der Monoanhydride der allgemeinen Formel III mit R⁵ = C₁-C₆-Alkylrest soll am Beispiel des Monoanhydrids des Diethylentriaminpentaessigsäure-ethylesters, ausgehend vom Monoethylester der DTPA (J. Pharm. Sci. 68, 1979, 194), beschrieben werden:

Die Herstellung der Monoanhydride der allgemeinen Formel III mit R⁵ = H erfolgt nach dem Fachmann bekannten Methoden (vgl. auch den experimentellen Teil) durch partielle Verseifung der entsprechenden Bisanhydride, wobei die dabei entstehenden Monoanhydride nicht isoliert werden müssen.

### N³-(2,6-Dioxomorpholinoethyl)-N⁶-(ethoxycarbonylmethyl)-3,6-diazaoctandisäure

Eine Suspension von 21,1 g (50 mMol N³,N⁶-Bis-(carboxymethyl)-N⁹-(ethoxycarbonylmethyl)-3,6,9-triazaundecandisäure in 250 ml Essigsäureanhydrid läßt man nach Zugabe von 42,2 ml Pyridin drei Tage bei Raumtemperatur rühren. Dann saugt man den Niederschlag ab, wäscht ihn dreimal mit je 50 ml Essigsäureanhydrid und verrührt ihn anschließend mehrere Stunden mit absolutem Diethylether. Nach Absaugen, Waschen mit absolutem Diethylether und Trocknen im Vakuum bei 40 °C erhält man 18,0 g (= 89 % der Theorie) eines weißen Pulvers vom Schmelzpunkt 195-196 °C.

Analyse (bezogen auf wasserfreie Substanz):
- berechnet:: C 47,64 H 6,25 N 10,42
- gefunden:: C 47,54 H 6,30 N 10,22

Die Umsetzung der Säureanhydride zu den Amiden wird in flüssiger Phase durchgeführt. Geeignete Reaktionsmedien sind beispielsweise Wasser, dipolare aprotische Lösungsmittel wie Acetonitril, N-Methylpyrrolidon, Dimethylformamid, Dimethylacetamid und dergleichen oder Gemische derselben. Die Reaktionstemperaturen liegen zwischen ca. 0 °C - 100 °C, wobei Temperaturen von 20 °C - 80 °C bevorzugt sind. Die Reaktionszeiten liegen zwischen 0,5 Stunden und 2 Tagen, vorzugsweise zwischen 1 Stunde und 36 Stunden.

Die Herstellung der Ester der allgemeinen Formel II erfolgt in bekannter Weise, z.B. nach den in R.A. Guilmette et al., J. Pharm. Sci. 68, 194 (1979) beschriebenen Verfahren.

Die Aminolyse der Ester erfolgt in flüssiger Phase, z.B. in einem geeigneten höhersiedenden Lösungsmittel wie Dimethylformamid, Dimethylacetamid oder Dimethylsulfoxid. Die Reaktionstemperaturen liegen bei etwa 20 °C - 200 °C, wobei Temperaturen von 100 °C - 180 °C bevorzugt sind. Die Reaktionszeiten liegen zwischen 2 Stunden und 2 Tagen, wobei Reaktionszeiten zwischen 4 Stunden und 36 Stunden bevorzugt sind.

Darüber hinaus können alle dem Fachmann bekannten Methoden zur Umwandlung von Carboxylgruppen in Amidgruppen zur Synthese der erfindungsgemäßen Komplexbildner der allgemeinen Formel I herangezogen werden, so z.B. die Methode nach Krejcarek und Tucker, Biochem. Biophys. Res. Commun. 77, 581 (1977) über gemischte Anhydride. Weitere Methoden zur Umwandlung aktivierter Carbonylgruppen sind z.B. in J. Nucl. Med. 24, 1158 (1983), Bioconjugate Chem. 1, 65 (1990), Org. Prep. Proc. Int. 7, 215 (1975), Adv. Org. Chem. Part B, 472 und Fieser, Reagents for Organic Syntheses 10, 142 (Carbodiimid) beschrieben.

Die Herstellung der als Ausgangssubstanzen für die C-substituierten (d.h. einer der Substituenten Z¹ und Z² ≠ Wasserstoff) Anhydride oder Ester der allgemeinen Formeln II und III benötigten Verbindungen erfolgt dadurch, daß man in an sich bekannter Weise Verbindungen der allgemeinen Formel V 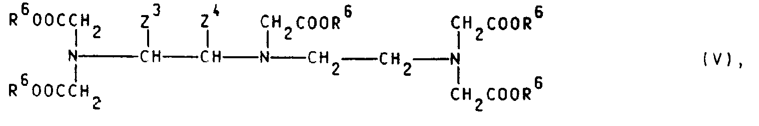
worin
- R⁶: für eine Säureschutzgruppe,
- Z³ und Z⁴: jeweils für ein Wasserstoffatom oder den Rest
-(CH₂)ₘ(-C₆H₄)_{q}-OH, mit der Maßgabe, daß einer der Substituenten Z³ und Z⁴ für ein Wasserstoffatom und der andere für den angegebenen Rest steht,

stehen,
in eine Verbindung mit dem für Z¹ und Z² angegebenen Rest umwandelt und die Säureschutzgruppen R⁶ abspaltet.

Als Säureschutzgruppen R⁶ kommen niedere Alkyl-, Aryl- und Aralkylgruppen, beispielsweise die Methyl-, Ethyl-, Propyl-, n-Butyl-, t-Butyl-, Phenyl-, Benzyl-, Diphenylmethyl-, Triphenylmethyl-, bis(p-Nitrophenyl)-methylgruppe, sowie Trialkylsilylgruppen infrage.

Die Abspaltung der Schutzgruppen R⁶ erfolgt nach den dem Fachmann bekannten Verfahren [z.B. E. Wünsch, Methoden der Org. Chemie (Houben-Weyl), Bd. XV/1, 4. Aufl. 1974, S 315 ff] beispielsweise durch Hydrolyse, Hydrogenolyse oder alkalische Verseifung der Ester mit Alkali in wäßrig-alkoholischer Lösung bei Temperaturen von 0 bis 50 °C. Zur Abspaltung der für die vorliegenden Reaktionen besonders vorteilhaften t-Butylester werden organische oder anorganische Säuren verwendet: Die in einem geeigneten wasserfreien organischen Lösungsmittel gelöste Esterverbindung, vorzugsweise jedoch die gepulverte Trockensubstanz, wird entweder mit Halogenwasserstoff-Lösung in Eisessig, mit Trifluoressigsäure oder auch Bortrifluorid-diethyletherat in Eisessig versetzt und bei Temperaturen von -10 °C bis 60 °C, vorzugsweise bei Raumtemperatur, abgespalten.

Die als Edukte für die Herstellung der erfindungsgemäßen Komplexverbindungen dienenden Verbindungen der allgemeinen Formel V sind bekannt (DOS 3 710 730 und dort zitierte Literatur) oder können analog der dort beschriebenen Herstellungsvorschriften synthetisiert werden.

Für die Umsetzung der bekannten aliphatischen oder aromatischen Hydroxyverbindungen zu den entsprechenden Aryl-alkyl- bzw. Dialkylethern steht eine Reihe von dem Fachmann bekannten Literaturmethoden zur Verfügung (z.B. J. March, Advanced Organic Chemistry, third edition 1985, S. 342 ff).

Dazu werden die Verbindungen der Formel V, wobei R⁶ für eine alkali-stabile Säureschutzgruppe steht, in einem polar aprotischen Lösungsmittel, wie z.B. Tetrahydrofuran, Dimethoxyethan oder Dimethylsulfoxid, gelöst und mit einer Base, wie z.B. Natriumhydrid, Natriumhydroxid oder Alkali- oder Erdalkalicarbonaten, bei Temperaturen zwischen -30 °C und dem Siedepunkt des jeweiligen Lösungsmittels versetzt, vorzugsweise jedoch zwischen 0 °C und 60 °C.

Dazu wird eine Verbindung der allgemeinen Formel VI

Y-(CH₂)ₙ-(C₆H₄)ₗ-(O)ᵣ-R (VI)

gegeben, wobei Y für ein Nucleofug, wie z.B. Cl, Br, I, CH₃-C₆H₄SO₃ oder CF₃SO₃, steht und die übrigen Indices die gleiche Bedeutung haben wie in der allgemeinen Formel I.

Die Reaktionszeiten betragen je nach sterischer Hinderung der beteiligten Reste 30 Minuten bis 8 Stunden.

Alternativ zu den oben beschriebenen Reaktionsbedingungen können sowohl Aryl-alkyl- als auch Dialkylether sehr vorteilhaft durch Phasen-Transfer-Katalyse (Starks and Liotta, Phase Transfer Catalysis, Academic Press, N.Y. 1978, S. 128-138) hergestellt werden.

Dazu wird die Reaktion in einem Zweiphasengemisch aus wäßriger Base, vorzugsweise 30 %iger Natronlauge, und einem mit Wasser nicht mischbaren organischen aprotischen Lösungsmittel durchgeführt. Als Phasen-Transferkatalysatoren kommen die dem Fachmann bekannten Verbindungen infrage, vorzugsweise jedoch Tetraalkylammonium- oder Tetraalkylphosphoniumsalze.

Will man Verbindungen der allgemeinen Formel I mit k, n, l und r = O und R in der Bedeutung eines Wasserstoffatoms synthetisieren, so ist es möglich, von der entsprechenden unsubstituierten Aminosäure (z.B. Phenylalanin) ausgehend, die Synthese analog den literaturbekannten Methoden durchzuführen.

Soll jedoch eine Reihe analoger Verbindungen synthetisiert werden, empfiehlt sich die Darstellung der in DOS 3 710 730 beschriebenen Phenolderivate und die reduktive Entfernung der Phenolfunktion nach den dem Fachmann bekannten Literaturverfahren. Genannt sei vor allem die Reduktion von Aryl-diethylphosphaten mit Titan, die sich auch in Gegenwart von Estergruppen sehr vorteilhaft durchführen läßt [S.C. Welch et al., J. Org. Chem. 43, 4797-99 (1978) und dort zitierte Literatur]. Hierbei wird zunächst aus dem phenolischen Edukt durch Umsetzung mit Phosphorsäure-diethylesterchlorid in 70 - bis 100 %iger Ausbeute das entsprechende Aryl-diethylphosphat gebildet, vorzugsweise durch Verwendung von Natriumhydrid als Base in einem polar aprotischen Lösungsmittel. Anschließend wird die Reduktion mit frisch hergestelltem Titanmetall durchgeführt. Vorzugsweise wird zur Herstellung von hochaktivem Titan wasserfreies Titan(III)-chlorid durch Magnesium oder Kalium in wasserfreiem Tetrahydrofuran unter Inertgas reduziert.

Zu einer solchen Mischung wird das oben beschriebene Diethylphosphat zugegeben und 2 bis 24 Stunden, vorzugsweise 6 bis 16 Stunden, unter Rückfluß erhitzt. Nach Beendigung der Reaktion wird gegebenenfalls chromatographisch aufgearbeitet. Ebenfalls anwendbar ist die Palladium-katalysierte Reduktion der entsprechenden Aryl-Triflate nach S. Cacchi et al., Tetr. Lett. 27, 5541-44 1986).

Die letztendlich so erhaltenen Verbindungen der allgemeinen Formel I mit X in der Bedeutung eines Wasserstoffatoms stellen Komplexbildner dar. Sie können isoliert und gereinigt werden oder ohne Isolierung in Metallkomplexe der allgemeinen Formel I mit mindestens zwei der Substituenten X in der Bedeutung eines Metallionenäquivalents überführt werden.

Die Herstellung der erfindungsgemäßen Metallkomplexe erfolgt in der Weise, wie sie in der Deutschen Offenlegungsschrift 34 01 052 offenbart worden ist, indem man das Metalloxid oder ein Metallsalz (beispielsweise das Nitrat, Acetat, Carbonat, Chlorid oder Sulfat) des Elements der Ordnungszahlen 21-29, 42, 44, 57-83 in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome durch Kationen anorganischer und/oder organischer Basen oder Aminosäuren substituiert.

Die Neutralisation eventuell noch vorhandener freier Säuregruppen erfolgt mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Eine andere Möglichkeit, zu neutralen Komplexverbindungen zu kommen, besteht darin, die verbleibenden Säuregruppen im Komplex ganz oder teilweise in zum Beispiel Ester oder Amide zu überführen. Dies kann durch nachträgliche Reaktion am fertigen Komplex geschehen (z.B. durch erschöpfende Umsetzung der freien Carboxy-Gruppen mit Dimethylsulfat).

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern (wie zum Beispiel Diethylentriaminpentaessigsäure) oder - falls erforderlich - Elektrolyte wie zum Beispiel Natriumchlorid oder - falls erforderlich - Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween^{(R)}, Myrj^{(R)} und/oder Aromastoff(en) zur Geschmackskorrektur (zum Beispiel ätherischen Ölen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 0, 1 »Mol - 1 Mol/l des Komplexsalzes und werden in der Regel in Mengen von 0,1 »Mol - 5 mMol/kg dosiert. Sie sind zur enteralen und parenteralen Applikation bestimmt. Die erfindungsgemäßen Komplexverbindungen kommen zur Anwendung.
1. für die NMR- und Röntgen-Diagnostik in Form ihrer Komplexe mit den Ionen der Elemente mit den Ordnungszahlen 21 - 29, 42, 44 und 57-83;
2. für die Radiodiagnostik und Radiotherapie in Form ihrer Komplexe mit den Radioisotopen der Elemente mit den Ordnungszahlen 21, 27, 29, 31, 32, 37-40, 43, 49, 62-64, 70, 75 und 77.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100 bis 1000fach besser wasserlöslich sein als für die NMR-Spektroskopie. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,0001 - 5 mMol/kg Körpergewicht, vorzugsweise 0,005 - 0,5 mMol/kg Körpergewicht des erfindungsgemäßen Komplexes, dosiert. Bei einer intravenösen Injektion finden wäßrige Formulierungen der Konzentration 50 »mol/l bis 2 mol/l, vorzugsweise 100 mmol/l bis 1 mol/l, Verwendung. Eine rektale sowie orale Anwendung wird vorzugsweise mit Lösungen der Konzentration 0,1 mmol bis 100 mmol/l oder als Feststoff im entsprechenden Konzentrationsbereich durchgeführt. Die applizierten Volumina sind je nach diagnostischer Fragestellung zwischen 5 ml und 2 l. Details der Anwendung werden zum Beispiel in H.J. Weinmann et al., Am. J. of Roentgenology 142, 619 (1984) diskutiert.

Besonders niedrige Dosierungen (unter 1 mg/kg Körpergewicht) von organspezifischen NMR-Diagnostika sind zum Beispiel zum Nachweis von Tumoren und von Herzinfarkt einsetzbar.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z. B. in "Radiotracers for Medical Applications", CRC-Press, Boca Raton, Florida, beschrieben.

Eine weitere bildgebende Methode mit Radioisotopen ist die Positronen-Emissions-Tomographie, die positronenemittierende Isotope wie z.B. ⁴³Sc, ⁴⁴Sc, ⁵²Fe, ⁵⁵Co und ⁶⁸Ga verwendet (Heiss, W.D.; Phelps, M.E.; Positron Emission Tomography of Brain, Springer Verlag Berlin, Heidelberg, New York 1983).

Die erfindungsgemäßen Verbindungen können auch in der Radioimmuno- oder Strahlentherapie verwendet werden. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Geeignete β-emittierende Ionen sind zum Beispiel ⁴⁶Sc, ⁴⁷Sc, ⁴⁸Sc, ⁷²Ga, ⁷³Ga und ⁹⁰Y. Geeignete geringe Halbwertzeiten aufweisende α-emittierende Ionen sind zum Beispiel ²¹¹Bi, ²¹²Bi, ²¹³Bi und ²¹⁴Bi, wobei ²¹²Bi bevorzugt ist. Ein geeignetes Photonen- und Elektronenemittierendes Ion ist ¹⁵⁸Gd, das aus ¹⁵⁷Gd durch Neutroneneinfang erhalten werden kann.

Ist das erfindungsgemäße Mittel zur Anwendung in der von R.L. Mills et al. [Nature Vol. 336, (1988), S. 787] vorgeschlagenen Variante der Strahlentherapie bestimmt, so muß sich das Zentralion von einem Mößbauer-Isotop wie beispielsweise ⁵⁷Fe oder ¹⁵¹Eu ableiten.

Bei der in-vivo-Applikation der erfindungsgemäßen therapeutischen Mittel können diese zusammen mit einem geeigneten Träger wie zum Beispiel Serum oder physiologischer Kochsalzlösung und/oder zusammen mit einem anderen Protein wie zum Beispiel Human Serum Albumin verabreicht werden. Die Dosierung ist dabei abhängig von der Art der zellulären Störung, dem benutzten Metallion und der Art der Methode, z.B. Brachytherapie.

Die erfindungsgemäßen therapeutischen Mittel werden parenteral, vorzugsweise i.v. appliziert.

Details der Anwendung von Radiotherapeutika werden z.B. in R.W. Kozak et al. TIBTEC, Oktober 1986, 262, diskutiert.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zu zum Beispiel Meglumin-Diatrizoat in Mengen von 0,1 - 5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler - "Einführung in die Röntgendiagnostik", G. Thieme, Stuttgart, New York (1977) diskutiert.

Insgesamt ist es gelungen, neue Komplexbildner, Metallkomplexe und Metallkomplexsalze zu synthetisieren, die neue Möglichkeiten in der diagnostischen und therapeutischen Medizin erschließen. Vor allem die Entwicklung neuartiger bildgebender Verfahren in der medizinischen Diagnostik läßt diese Entwicklung wünschenswert erscheinen.

Die nachfolgenden Beispiele dienen zur näheren Erläuterung des Erfindungsgegenstands.

### Beispiel 1

a) **3,6-Bis(carboxymethyl)-9-(10-carboxydecylcarbamoylmethyl)-3,6,9-triazaundecandisäure**
   15,33 g (38 mmol) N³-(2,6-Dioxomorpholinoethyl)-N⁶-(ethoxycarbonylmethyl)-3,6-diazaoctandisäure (Beispiel 13a der EP 0331 616) werden in Dimethylformamid (DMF) suspendiert und im Eisbad mit 21,07 ml (152 mmol) Triethylamin und 7,65 g (38 mmol) 11-Aminoundecansäure versetzt. Man läßt die Temperatur auf Raumtemperatur ansteigen und rührt die Reaktionslösung über Nacht. Nach beendeter Reaktion wird die eventuell noch leicht trübe Lösung filtriert und das Filtrat im Vakuum eingedampft. Der Rückstand wird mit Ether ausgerührt und anschließend aus Wasser umkristallisiert (17,8 g). Der Ethylester wird in 2N NaOH gelöst und nach 3 Stunden durch Zugabe von Amberlite^{R} IR 120 (H⁺) auf pH 7 gestellt. Der Ionenaustauscher wird abgesaugt, das Filtrat gefriergetrocknet (20,1 g) und anschließend in H₂O/Methanol (2:1) über 100 ml Amberlite^{R} IR 120 (H⁺) gegeben und das saure Eluat eingeengt und aus Wasser umkristallisiert.
   Ausbeute: 13,0 g (59,3 %)
   Fp. 168°C
   Analyse (bezogen auf wasserfreie Substanz) :
   - ber.:: C 52,07 H 7,69 N 9,72
   C 51,98 H 7,61 N 9,75
b) **Gadoliniumkomplex der** **3,6-Bis(carboxymethyl)-9-(10-carboxydecylcarbamoylmethyl)-3,6,9-triazaundecandisäure**
   8,65 g (15 mmol) der in Beispiel 1a beschriebenen Komplexbildnersäure werden in 200 ml H₂O suspendiert und mit 2,72 g (7,5 mmol) Gd₂O₃ versetzt und 60 Minuten auf dem Wasserbad bei 90°C gehalten. Die Lösung wird über Membranfilter filtriert, eingefroren und gefriergetrocknet.
   Ausbeute: 11,9 g (quantitativ)
   H₂O-Gehalt: 9,2 % (Karl-Fischer)
   Analyse (bezogen auf wasserfreie Subztanz) :
   - ber.:: C 41.08 H 5,65 Gd 21,52 N 7,67
   C 41,12 H 5,69 Gd 21,37 N 7,59
c) **Europiumkomplex der** **3,6-Bis(carboxymethyl)-9-(10-carboxydecylcarbamoylmethyl)-3,6,9-triazaundecandisäure**
   In analoger Weise erhält man aus der in Beispiel 1a beschriebenen Komplexbildnersäure mit Europiumoxid, Eu₂O₃, in quantitativer Ausbeute die Titelverbindung als weißes Pulver mit einem uncharakteristischen Zersetzungspunkt.
   Analyse (bezogen auf wasserfreie Subztanz) :
   - ber.:: C 40,52 H 5,58 N 7,56 Eu 22,58
   C 40,60 H 5,65 N 7,63 Eu 22,51
d) **Eisen-III-komplex der** **3,6-Bis(carboxymethyl)-9-(10-carboxydecylcarbamoylmethyl)-3,6,9-triazaundecandisäure**
   In analoger Weise erhält man aus der in Beispiel 1a beschriebenen Komplexbildnersäure mit Eisen-III-oxid, Fe₂O₃, in quantitativer Ausbeute die Titelverbindung als braunes Pulver mit einem uncharakteristischen Zersetzungspunkt.
   Analyse (bezogen auf wasserfreie Subztanz) :
   - ber.:: C 63,19 H 9,33 N 11,79 Fe 11,75
   C 63,04 H 9,42 N 11,68 Fe 11,79
e) **Wismut-III-komplex der** **3,6-Bis-(carboxymethyl)-9-(10-carboxydecylcarbamoylmethyl)-3,6,9-triazaundecandisäure**
   In analoger Weise erhält man aus der in Beispiel 1a beschriebenen Komplexbildnersäure mit Wismut-III-oxid, Bi₂O₃, in quantitativer Ausbeute die Titelverbindung als braunes Pulver mit einem uncharakteristischen Zersetzungspunkt.
   Analyse (bezogen auf wasserfreie Subztanz) :
   - ber.:: C 47,79 H 7,06 N 8,92 Bi 33,26
   C 47,83 H 7,11 B 8,88 Bi 33,10

### Beispiel 2

a) **6-Carboxymethyl-9-(4-carboxymethyl-phenyl)-carbamoylmethyl-3-ethoxycarbonylmethyl-3,6,9-triazaundecandisäure**
   Zu einer Suspension von 8,07 g (20 mmol) N³-(2,6-Dioxomorpholinoethyl)-N⁶-(ethoxy-carbonylmethyl)-3,6-diazaoctandisäure in 100 ml Dimethylformamid gibt man bei 45°C unter Rühren 3,02 g (20 mmol) p-Aminophenylessigsäure, rührt anschließend über Nacht bei Raumtemperatur, dampft das Lösungsmittel im Vakuum weitgehend ab und rührt den Rückstand mit 100 ml Methyl-tert.butyl-ether, saugt ab und trocknet den Rückstand im Vakuum bei 50°C. Das so erhaltene Rohprodukt wird mit 10 ml Wasser suspendiert. Man versetzt mit 11N Natronlauge bis zum pH 7, wobei die Substanz in Lösung geht. Zur Lösung gibt man 10 g Kieselgel, dampft die Suspension am Rotationsverdampfer ein und gibt den Rückstand auf eine Säule mit 700 g Kieselgel, die mit einer Mischung aus Chloroform-Methanol-Eisessig-Wasser (18-10-4-4) aufgezogen wurde. Man eluiert mit dem gleichen Lösungsmittel und isoliert nach Eindampfen des Eluats 8,3 g öliges Produkt, das zur Entfernung von Natriumionen in 75 ml Wasser gelöst und auf eine Säule aus 75 ml Kationenaustauscher IR 120 gegeben wird. Die Säule wird mit 150 ml Wasser eluiert und die wässrige Lösung im Vakuum eingedampft. Den Rückstand rührt man mit 50 ml Diethylether, saugt ab, trocknet im Vakuum und erhält 6,2 g der Titelverbindung als weißes Pulver.
   Analyse (bezogen auf wasserfreie Subztanz) :
   - ber.:: C 51,98 H 6,18 N 10,10
   C 51,81 H 6,33 N 10,18
b) **3,6-Bis(carboxymethyl)-9-(4-carboxymethyl-phenyl)-carbamoylmethyl-3,6,9-triazaundecandisäure**
   Zu einer Lösung von 5 g des nach Teil a) hergestellten Esters in 20 ml Wasser gibt man 90 ml 1N Natronlauge, läßt 3 Stunden bei Raumtemperatur stehen und gibt in die Lösung unter Rühren ca. 200 ml Kationenaustauscher IR 120, die Lösung hat danach einen pH-Wert von 2,3. Nach Filtrieren wird die Lösung gefriergetrockent. Man erhält 3,90 g der Titelverbindung als weißes Pulver.
   Analyse (bezogen auf wasserfreie Subztanz) :
   - ber.:: C 50,19 H 5,74 N 10,64
   C 50,03 H 5,71 N 10,72
c) **Gadoliniumkomplex der** **3,6-Bis(carboxymethyl)-9-(4-carboxymethyl-phenyl)-carbamoylmethyl-3,6,9-triazaundecandisäure**
   1,053 g (2 mmol) der nach Teil b) hergestellten Substanz werden in 50 ml Wasser mit 362 mg (1 mmol) Gadoliniumoxid 1 Stunde auf 80-85°C erhitzt. Dabei geht das Oxid in Lösung. Man filtriert durch ein 0,1 » Membranfilter und isoliert die Substanz durch Gefriertrocknen. Man erhält 1,35 g der Titelverbindung als weißes Pulver.
   Analyse (bezogen auf wasserfreie Subztanz) :
   - ber.:: C 38,82 H 4,00 Gd 23,10 N 8,23
   C 38,57 H 4,40 Gd 22,95 N 8,33

### Beispiel 3

a) **6-Carboxymethyl-9-[4-(carboxyisopropyl)-phenyl]-carbamoylmethyl-3-ethoxycarbonylmethyl-3,6,9-triozaundecandisäure**
   In Analogie zu Beispiel 2a erhält man aus N³-(2,6-Dioxomorpholinoethyl)-N⁶-(ethoxy-carbonylmethyl)-3,6,-diazaoctandisäure und α,α-Dimethyl-p-aminophenylessigsäure die Titelverbindung.
   Analyse (bezogen auf wasserfreie Subztanz) :
   - ber.:: C 53,60 H 6,57 N 6,92
   C 53,44 H 6,88 N 9,49
b) **3,6-Bis(carboxymethyl)-9-[4-(carboxyisopropyl)-phenyl]****-carbamoylmethyl-3,6,9-triazaundecandisäure**
   In Analogie zu Beispiel 2b erhält man durch alkalische Verseifung von 6-Carboxymethyl-9-[4-(carboxyisopropyl)-phenyl]-carbamoylmethyl-3-ethoxycarbonylmethyl-3,6,9-triazaundecandisäure die Titelverbindung.
   Analyse (bezogen auf wasserfreie Subztanz) :
   - ber.:: C 51,98 H 6,18 N 10,10
   C 51,79 H 6,35 N 10,01
c) **Gadoliniumkomplex der** **3,6-Bis(carboxymethyl)-9-[4-(carboxyisopropyl)-phenyl]-carbamoylmethyl-3,6,9-triazaundecandisäure**
   In Analogie zu Beispiel 2c erhält man die Titelverbindung durch Komplexierung des Liganden mit Gadoliniumoxid.

Analyse (bezogen auf wasserfreie Subztanz) :
- ber.:: C 40,67 H 4,41 Gd 22,19 N 7,90
C 40,47 H 4,60 Gd 22,01 N 7,80

### Beispiel 4

a) **3,6-Bis(carboxymethyl)-9-(11-carboxy-1-n-hexyl-undecylcarbamoylmethyl)-3,6,9-triazaundecandisäure**
   15,33 g (38 mmol) N³-(2,6-Dioxomorpholinoethyl)-N⁶-(ethoxycarbonylmethyl)-3,6-diazaoctandisäure (Beispiel 13a der EP 0331 616) werden in 100 ml Dimethylformamid suspendiert und im Eisbad mit 21,07 ml (152 mmol) Triethylamin und 11,36 g (38 mmol) 12-Aminooctadecansäure versetzt. Nach Rühren über Nacht bei Raumtemperatur engt man im Vakuum ein und rührt den Rückstand mit 500 ml Diethylether aus. Man saugt ab und löst das getrocknete Reaktionsprodukt in 50 ml 2n-Natronlauge. Nach dreistündigem Rühren neutralisiert man mit verd. Salzsäure, engt im Vakuum zur Trockene ein und kristallisiert aus Wasser um. Man erhält 15,64 g (61 % d. Th.) eines grauen Pulvers.
   Analyse (bezogen auf wasserfreie Subztanz) :
   - ber.:: C 56,95 H 8,66 N 8,30
   C 56,87 H 8,77 N 8,35
b) **Gadoliniumkomplex der** **3,6-Bis(carboxymethyl)-9-(11-carboxy-1-n-hexyl-undecylcarbamoyl-methyl)-3,6,9-triazaundecandisäure**
   10,12 g (15 mmol) der vorstehend beschriebenen Verbindung werden in 200 ml Wasser suspendiert und mit 2,72 g (7,5 mmol) Gadoliniumoxid versetzt. Man erhitzt 60 Minuten auf 90°C, läßt auf Raumtemperatur abkühlen, filtriert und lyophilisiert die Lösung. Man erhält 12,43 g (quantitativ) der Titelverbindung als weißes Pulver.

Analyse (bezogen auf wasserfreie Subztanz) :
- ber.:: C 46,36 H 6,69 N 6,76 Gd 18,97
C 46,31 H 6,75 N 6,68 Gd 18,95

Die Substanz bildet in wässriger Lösung Assoziate mit Human-Serum-Albumin.

### Beispiel 5

**a)** **3,6-Bis(carboxymethyl)-9-(10-carbethoxydecylcarbamoylmethyl)-3,6,9-triazaundecandisäure**
   17,85 g (50 mmol) 1,5-Bis(2,6-dioxomorpholino)-3-carboxymethyl-3-azapentan werden in 200 ml Dimethylformamid gerührt und mit 9 ml (50 mmol) Wasser versetzt. Man erwärmt die Lösung 3 Stunden auf 70 °C, kühlt auf 0 °C ab und gibt 36,07 ml (260 mmol) Triethylamin und 10,8 g (50 mmol) 11-Aminoundecansäureethylester zu. Nach Rühren über Nacht bei Raumtemperatur wird die Lösung im Vakuum zur Trockne eingeengt. Der Rückstand wird in 100 ml Wasser aufgenommen und die Lösung tropfenweise mit konz. Salzsäure bis zur bleibenden Trübung versetzt. Nach Rühren über Nacht im Eisbad wird der feinkristalline Niederschlag abgesaugt, mit Wasser gewaschen und bei 50 °C im Vakuum getrocknet. Man erhält 14,9 g (65 % der Theorie) der Titelverbindung als weißes Pulver vom Schmelzpunkt 155 - 157 °C.
   Analyse (bezogen auf wasserfreie Subztanz) :
   - ber.:: C 53,63 H 8,00 N 9,27
   C 53,58 H 8,22 N 9,41
**b)** **Gadoliniumkomplex der** **3,6-Bis(carboxymethyl)-9-(10-carbethoxydecylcarbamoylmethyl)-3,6,9-triazaundecandisäure**
   9,08 g (15 mmol) der in Beispiel 5a) beschriebenen Verbindung werden in 200 ml Wasser suspendiert, mit 2,72 g (7,5 mmol) Gd₂O₃ versetzt und unter Rühren bei Raumtemperatur zur klaren Lösung gebracht. Durch Gefriertrocknung erhält man den Gadoliniumkomplex in quantitativer Ausbeute als weißes Pulver.

Analyse (bezogen auf wasserfreie Subztanz) :
- ber.:: C 42,73 H 5,98 N 7,38 Gd 20,72
C 42,80 H 5,87 N 7,51 Gd 20,63

### Beispiel 6

**a)** **1,5-Bis(2,6-dioxomorpholino)-2-(4-ethoxybenzyl)-3-carboxymethyl-3-azapentan**
   52,8 g (100 mmol) 3,6,9-Triaza-3,6,9-tris(carboxymethyl)-4-(4-ethoxybenzyl)undecandisäure, hergestellt nach EP-Publikations-Nr. 0 405 704 [Beispiel 8b)], werden in 75 ml Essigsäureanhydrid suspendiert. Man gibt 38 ml Pyridin zu und erwärmt 3 Stunden auf 50 °C. Nach Abkühlen auf Raumtemperatur und Rühren über Nacht wird der Niederschlag abgesaugt, dreimal mit je 200 ml Diethylether gewaschen und im Vakuum bei 30 °C getrocknet. Man erhält 40.3 g (82 % der Theorie) der Titelverbindung als weißes Pulver vom Schmelzpunkt 172 - 174 °C.
   Analyse
   - ber.:: C 56,21 H 5,95 N 8,55
   C 56,35 H 5,88 N 8,60
**b)** **3,6-Bis(carboxymethyl)-4-(4-ethoxybenzyl]-9-(10-carboxydecylcarbamoylmethyl)-3,6,9-triazaundecandisäure und**
   **6,9-Bis(carboxymethyl)-4-(4-ethoxybenzyl)-3-(10-carboxydecylcarbamoylmethyl)-3,6,9-triazaundecandisäure**
   24,6 g (50 mmol) der nach Beispiel 6a) erhaltenen Verbindung werden in 200 ml Dimethylformamid gerührt und mit 9 ml (50 mmol) Wasser versetzt. Man erwärmt die Lösung 3 Stunden auf 70 °C, kühlt auf 0 °C ab und gibt 36,07 ml (260 mmol) Triethylamin und 10,0 g (50 mmol) 11-Aminoundecansäure zu. Nach Rühren über Nacht bei Raumtemperatur wird die Lösung im Vakuum zur Trockne eingeengt. Der Rückstand wird in 500 ml Wasser aufgenommen und tropfenweise mit konz. Salzsäure bis zur bleibenden Trübung versetzt. Nach Rühren über Nacht im Eisbad wird der Niederschlag abgesaugt, mit Wasser gewaschen und bei 50 °C im Vakuum getrocknet. Man erhält 21,3 g (60 % der Theorie) des Isomerengemischs als weißes Pulver vom Schmelzpunkt 184 - 186 °C.
   Analyse (bezogen auf wasserfreie Subztanz) :
   - ber.:: C 57,45 H 7,66 N 7,88
   C 57,38 H 7,80 N 7,95
**c) Gadoliniumkomplex der** **3,6-Bis(carboxymethyl)-4-(4-ethoxybenzyl)-9-(10-carboxydecylcarbamoylmethyl)-3,6,9-triazaundecandisäure und**
   **6,9-Bis(carboxymethyl)-4-(4-ethoxybenzyl)-3-(10-carboxydecylcarbamoylmethyl)-3,6,9-triazaundecandisäure**
   10,65 g (15mol) der in Beispiel 6b) beschriebenen Komplexbildner werden in 200 ml Wasser suspendiert und mit 2,72 g (7,5 mmol) Gd₂O₃ versetzt. Man erhitzt 1 Stunde auf 90 °C, gibt die Lösung über ein Membranfilter und isoliert die Komplexe durch Gefriertrocknung in quantitativer Ausbeute als weißes Pulver.

Analyse (bezogen auf wasserfreie Subztanz) :
- ber.:: C 47,20 H 5,94 N 6,48 Gd 18,18
C 47,15 H 5,73 N 6,39 Gd 18,30

### Beispiel 7

**a)** **3,6,9-Tris(carboxymethyl)-3,6,9-triazaundecandisäure-[N-methyl-N-(4-carboxyphenyl)]monoamid**
   15,33 g (38 mmol) N³-(2,6-Dioxomorpholinoethyl)-N⁶-(ethoxycarbonylmethyl)-3,6-diazaoctandisäure [Beispiel 13a) der EP 0 331 616] werden in 100 ml Dimethylformamid suspendiert und bei 0 °C mit 21 ml Triethylamin und 5,74 g (38 mmol) 4-N-Methylaminobenzoesäure versetzt. Nach Rühren über Nacht bei Raumtemperatur engt man im Vakuum zur Trockne ein und rührt den Rückstand mit 500 ml Diethylether aus. Man saugt ab und erhält nach Umkristallisieren aus Ethanol 11,9 g (60 % der Theorie) der Titelverbindung als weißes Pulver.
   Analyse
   - ber.:: C 50,28 H 5,56 N 10,66
   C 50,35 H 5,61 N 10,58
**b) Gadoliniumkomplex des** **3,6,9-Tris(carboxymethyl)-3,6,9-triazaundecandisäure[N-methyl-N-(4-carboxyphenyl]monoamids**
   7,88 g (15 mmol) der vorstehend beschriebenen Verbindung werden in 200 ml Wasser suspendiert und mit 2,72 g (7,5 mmol) Gd₂O₃ versetzt. Man erhitzt 60 Minuten auf 90 °C, läßt auf Raumtemperatur abkühlen und lyophilisiert die Lösung. Man erhält die Titelverbindung als weißes Pulver mit einem uncharakteristischen Zersetzungsprodukt in quantitativer Ausbeute.

Analyse (bezogen auf wasserfreie Subztanz) :
- ber.:: C 38,87 H 3,86 N 8,24 Gd 23,13
C 38,81 H 3,94 N 8,31 Gd 23,25

### Beispiele für die Darreichungsform

### Herstellung einer Lösung vom Gadoliniumkomplex der 3,6-Bis(carboxymethyl)-9-(10-carboxydecylcarbamoylmethyl)-3,6,9-triazaundecandisäure

365,43 g (0,5 mol) der in Beispiel 1b beschriebenen Verbindung werden unter Zugabe von 195,22 g (1 mol) N-Methylglucamin in 600 ml Wasser pro injectione (p.i.) gelöst. Nach Zugabe von 3,22 g Monohydrat des Calcium-Trinatriumsalzes der DTPA, CaNa₃ DTPA, und 1,21 g Trishydroxymethylaminomethan stellt man mit verd. Salzsäure auf pH 7,0 und füllt mit Wasser p.i. auf 1000 ml auf. Die Lösung wird ultrafiltriert, in Flaschen abgefüllt und hitzesterilisiert.

### Zusammensetzung eines Pulvers zur oralen Applikation

12,8 g (35 mmol) der in Beispiel 1b beschriebenen Verbindung
12,0 g Saccharose
0,5 g Polyoxyethylenpolyoxypropylen-Polymeres
0,001 g Aromastoffe

### Beispiel für eine in-vivo-NMR-Diagnostik

Einem Versuchstier (Ratte, Stamm Lew/Mol, ♀, 155 g) wurde 2 Wochen vor dem Versuch eine Zellsuspension des Brown-Pearce Tumors in die Leber injiziert. Bei der angewandten Methode bedarf es etwa 2 Wochen, bis der Tumor die für die Untersuchung erwünschte Größe von 0,5 - 1 cm³ erreicht hat.

Für den Versuch wurde das Tier narkotisiert (Rompun + Ketavet) und anschließend für die Applikation des Kontrastmittels ein Katheter in die Schwanzvene gelegt.

Das Imaging erfolgte in einem MRI-Experimentalgerät der Firma General Electric (Feldsträke 2 Tesla). Das erste Bild wurde ohne Kontrastmittel mit einer T₁-gewichteten Spin-Echo-Sequenz (TR = 400 msec, TE - 20 msec,Schichtdicke 3 mm) erstellt. Die Leber erscheint mit der erwarteten (normalen) Signalintensität; der Tumor ist isodens und nicht abgrenzbar. Danach erfolgte die Kontrastmittelgabe [Gadoliniumkomplex der 3,6-Bis(carboxymethyl)-9-(10-carboxydecylcarbamoylmethyl)-3,6,9-triazaundecandisäure, Beispiel 1b)] durch den Venenkatheter mit einer Dosis von 0,1 mmol Gd/kg (Konz. der Lösung 0,05 mmol/ml in 0,9 % NaCl, also einer z.B. bei dem Handelspräparat Magnevist^{(R)} klinisch üblichen Dosis), Bild Nr. 2 wurde 60 Minuten nach Kontrastmittelgabe unter sonst gleichen Bedingungen wie Bild Nr. 1 aufgenommen. Das Leberparenchym erscheint jetzt sehr hell, und man erkennt deutlich den Tumor, der wesentlich dunkler im Vergleich zum umliegenden Lebergewebe ist. Dies bedeutet, daß das Kontrastmittel in hohem Maße in der Leber angereichert wird, im Tumor hingegen nicht. Insofern bietet sich hier für die Diagnostik von Lebertumoren ein erheblicher Vorteil gegenüber dem bisher einzigen auf dem Markt befindlichen Kontrastmittel für die Kernspintomographie Magnevist^{(R)}.

Des weiteren zeigt sich anhand des sehr starken Enhancements des Magens im Bild Nr. 2, daß die Substanz offenbar auch in den Magen sezerniert wird. Dies bietet weitere diagnostische Möglichkeiten im Hinblick auf eine bessere Abgrenzung von Leber und Magen.

In einem weiteren Tier (Ratte, Stamm Lew/Mol, ♀, 200 g) wurde das gleiche Kontrastmittel oral mit einer p.o.-Sonde in einer Dosis von 0,5 mmol Gd/kg (Konzentration der Lösung 0,033 mmol Gd/ml, in 0,9 % NaCl) verabreicht. 30 Minuten nach Applikation wurde das Tier narkotisiert und unter gleichen Bedingungen untersucht wie im ersten Fall beschrieben. Bereits 45 Minuten (Bild 3) nach Applikation deutet sich ein leichter Signalanstieg in der Leber an. 180 Minuten nach Applikation (Bild 4) erkennt man ein deutliches Enhancement in der Leber, was darauf hindeutet, daß die Substanz nach oraler Applikation im Dünndarm resorbiert wird und zum großen Teil sofort wieder von der Leber aufgenommen wird. Dieser Befund deckt sich sehr gut mit den pharmakokinetischen Daten. Es folgt, daß also auch mit einer oralen Gabe des Kontrastmittels eine Lebertumordiagnostik möglich ist.

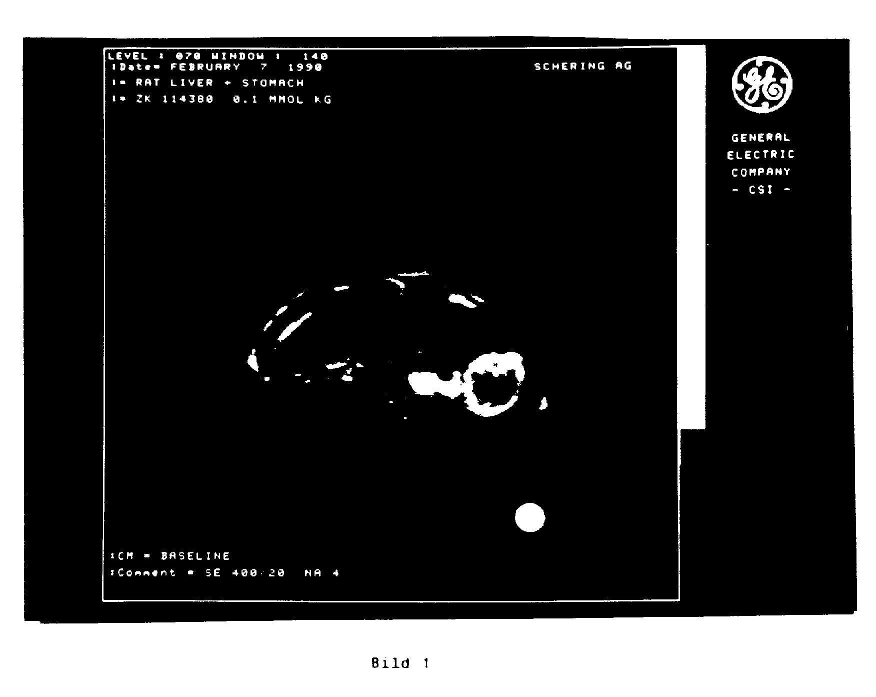

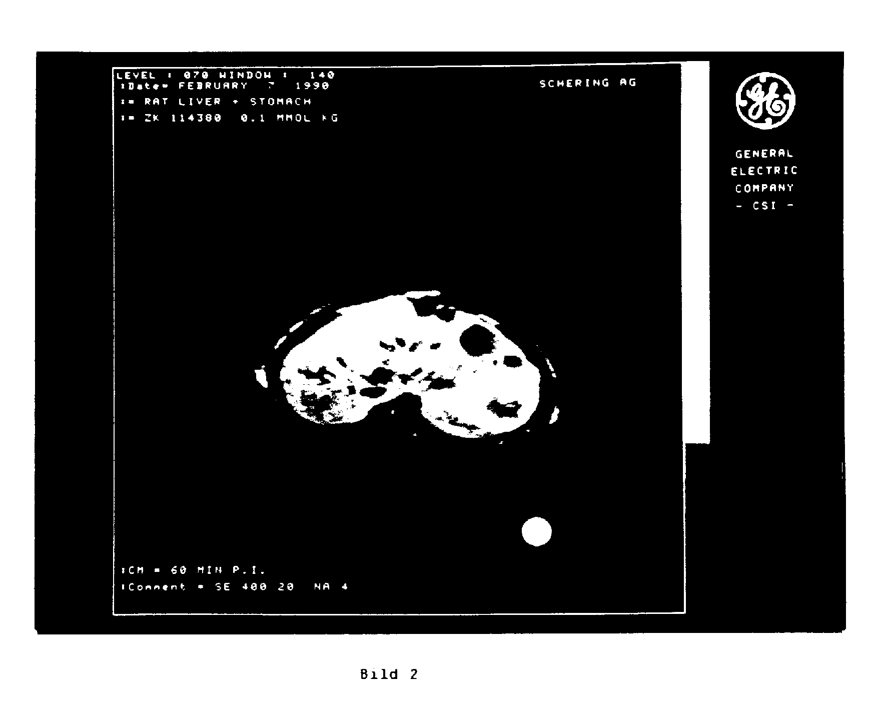

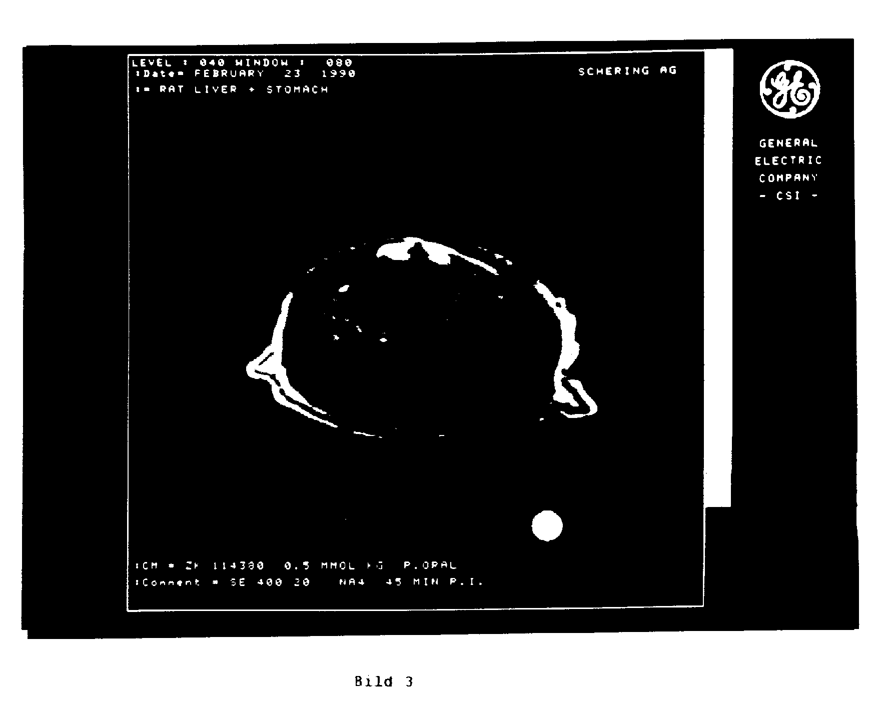

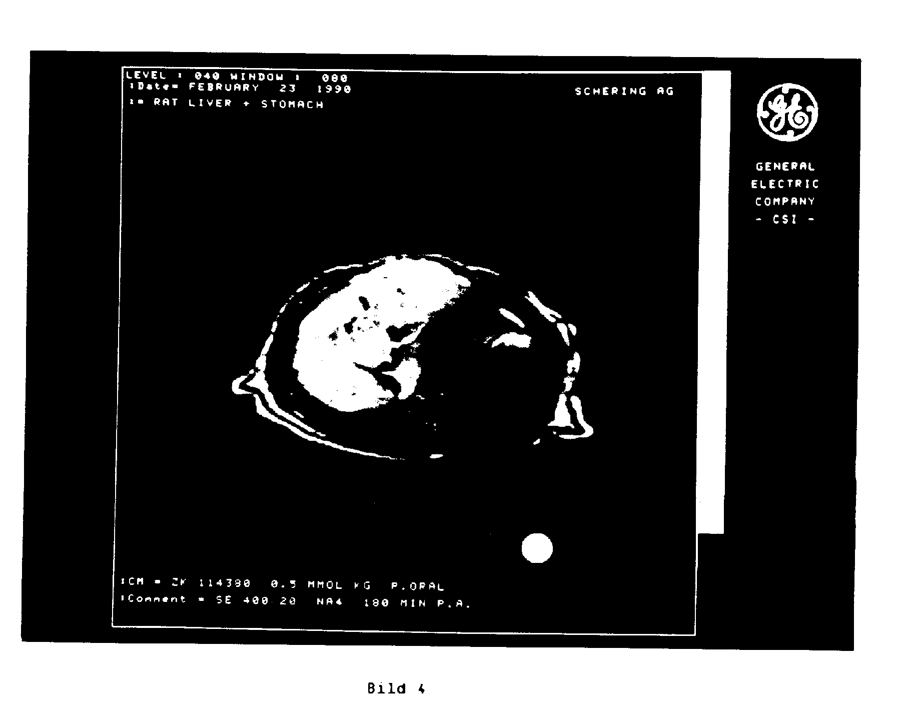

## Patentansprüche

1. Verbindungen der allgemeinen Formel I 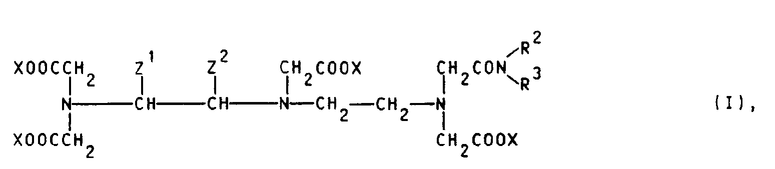 worin
Z¹ und Z² jeweils für ein Wasserstoffatom oder den Rest
-(CH₂)ₘ-(C₆H₄)_{q}-(O)ₖ-(CH₂)ₙ-(C₆H₄)ₗ-(O)ᵣ-R,
worin
m und n die Ziffern 0 - 20,
k, l, q und r die Ziffern 0 und 1 und
R ein Wasserstoffatom, einen gegebenenfalls OR¹-substituierten C₁-C₆-Alkylrest oder eine CH₂COOR¹-Gruppe mit R¹ in der Bedeutung eines Wasserstoffatoms, eines C₁-C₆-Alkylrestes oder einer Benzylgruppe bedeuten,
R² für eine durch eine gegebenenfalls mit einem C₁-C₆-Alkyl- oder Benzylrest veresterte Carboxyl- oder Sulfogruppe substituierte gesättigte, ungesättigte, gerad- oder verzweigtkettige oder cyclische Alkylgruppe mit bis zu 20 C-Atomen, Aryl- oder Aralkylgruppe,
R³ für ein Wasserstoffatom oder für eine gegebenenfalls durch eine gegebenenfalls mit einem C₁-C₆-Alkyl- oder Benzylrest veresterte Carboxyl- oder Sulfogruppe substituierte gesättigte, ungesättigte, gerad- oder verzweigtkettige oder cyclische Alkylgruppe mit bis zu 20 C-Atomen, Aryl- oder Aralkylgruppe,
X für ein Wasserstoffatom und/oder ein Metallionenäquivalent eines Elements der Ordnungszahlen 21-29, 31, 32, 37-40, 42-44, 49 oder 57-83 stehen, mit der Maßgabe, daß mindestens einer der Substituenten Z¹ und Z² für ein Wasserstoffatom steht, daß - wenn n und l jeweils für die Ziffer 0 stehen - k und r nicht gleichzeitig jeweils die Ziffer 1 bedeuten und daß gewünschtenfalls der Rest der Säuregruppen als Ester oder Amid vorliegt, sowie deren Salze mit anorganischen und/oder organischen Basen, Aminosäuren oder Aminosäureamide.

2. Verbindungen gemäß Anspruch 1, dadurch gekennzeichnet, daß mindestens zwei der Substituenten X Metallionenäquivalente mindestens eines Elements der Ordnungszahlen 21-29, 42, 44 oder 57-83 oder mindestens eines Radionuklids eines Elements der Ordnungszahlen 27, 29, 31, 32, 37-39, 43, 49, 62, 64, 70 oder 77 sind.

3. Verbindungen gemäß Anspruch 1, dadurch gekennzeichnet, daß Z¹ und Z² jeweils für ein Wasserstoff stehen.

4. Verbindungen gemäß Anspruch 1 , dadurch gekennzeichnet, daß Z¹ für ein Wasserstoffatom und Z² für den Rest -(CH₂)ₘ-(C₆H₄)_{q}-(O)ₖ-(CH₂)ₙ-(C₆H₄)ₗ-(O)ᵣ-R steht.

5. Verbindungen gemäß Anspruch 1, dadurch gekennzeichnet, daß Z² für ein Wasserstoffatom und Z¹ für den Rest
-(CH₂)ₘ-(C₆H₄)_{q}-(O)ₖ-(CH₂)ₙ-(C₆H₄)ₗ-(O)ᵣ-R
steht.

6. Verbindungen gemäß Anspruch 1, dadurch gekennzeichnet, daß Z¹ bzw. Z² für die Reste -CH₂-C₆H₄-OH, -CH₂-C₆H₄-OCH₃, -CH₂-C₆H₅, -CH₂-C₆H₄-O-CH₂-C₆H₄-OCH₃, -CH₂-O-CH₂-C₆H₅, -CH₂-C₆H₄-O-CH₂-COOH, -CH₂-C₆H₄-OC₂H₅, -CH₂-C₆H₄-OC₄H₉, -CH₂-C₆H₄-O-CH₂-C₆H₅ steht.

7. Verbindungen gemäß Anspruch 1, dadurch gekennzeichnet, daß R² bzw. R³ für den Methyl-, Ethyl-, Propyl-, Isopropyl-, Butyl-, Pentyl-, Hexyl-, Phenyl-, Benzyl-, 4-Carboxy-phenylen-, 1 -Cyclohexyl-1-carbonsäure-, 1-Cyclopentyl-1-carbonsäure-, 2-Carboxy-phenylen-, 11-Carboxy-1-undecyl-, 10-Carboxy-1-decyl-, 7-Carboxy-1-heptyl-, 6-Carboxy-1-hexyl-, 5-Carboxy-1-pentyl-, 4-Carboxy-benzyl-, 4-Carboxy-1-cyclohexylmethyl-, 4-Carboxymethyl-phenylen-, 4-(1,1-Dimethylcarboxymethylen)-phenylen-, 4-Sulfo-phenylen-, 10-Sulfo-1-decyl-, 8-Sulfo-1-octyl-, 17-Carboxy-7-heptadecyl-, 7-Carboxy-2-heptyl-, 1-Carboxy-4-phenyl-2-propyl-, 2-Carboxyethyl-, 4-Carboxy-4-but-1-enyl-Rest stehen.

8. Pharmazeutische Mittel enthaltend mindestens eine physiologisch verträgliche Verbindung nach Anspruch 1, gegebenenfalls mit den in der Galenik üblichen Zusätzen.

9. Verwendung von mindestens einer physiologisch verträglichen Verbindung gemäß Anspruch 1 für die Herstellung von Mitteln für die NMR-, Röntgen-, Radio-Diagnostik, Radio- oder Strahlen-Therapie.

10. Verwendung von mindestens einer physiologisch verträglichen Verbindung gemäß Anspruch 1 für die Herstellung von Mitteln für die NMR-Diagnostik des Magen-Darm-Traktes, des renalen und hepatobiliären Systems.

11. Verfahren zur Herstellung von Verbindungen der allgemeinen Formel I 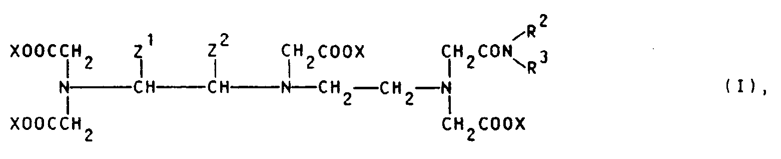 worin
Z¹ und Z² jeweils für ein Wasserstoffatom oder den Rest
-(CH₂)ₘ-(C₆H₄)_{q}-(O)ₖ-(CH₂)ₙ-(C₆H₄)ₗ-(O)ᵣ-R,
worin
m und n die Ziffern 0 - 20,
k, l, q und r die Ziffern 0 und 1 und
R ein Wasserstoffatom, einen gegebenenfalls OR¹-substituierten C₁-C₆-Alkylrest oder eine CH₂COOR¹-Gruppe mit R¹ in der Bedeutung eines Wasserstoffatoms, eines C₁-C₆-Alkylrestes oder einer Benzylgruppe bedeuten,
R² für eine durch eine gegebenenfalls mit einem C₁-C₆-Alkyl- oder Benzylrest veresterte Carboxyl- oder Sulfogruppe substituierte gesättigte, ungesättigte, gerad- oder verzweigtkettige oder cyclische Alkylgruppe mit bis zu 20 C-Atomen, Aryl- oder Aralkylgruppe,
R³ für ein Wasserstoffatom oder für eine gegebenenfalls durch eine gegebenenfalls mit einem C₁-C₆-Alkyl- oder Benzylrest veresterte Carboxyl- oder Sulfogruppe substituierte gesättigte, ungesättigte, gerad- oder verzweigtkettige oder cyclische Alkylgruppe mit bis zu 20 C-Atomen, Aryl- oder Aralkylgruppe,
X für ein Wasserstoffatom und/oder ein Metallionenäquivalent eines Elements der Ordnungszahlen 21-29, 31, 32, 37-40, 42-44, 49 oder 57-83 stehen, mit der Maßgabe, daß mindestens einer der Substituenten Z¹ und Z² für ein Wasserstoffatom steht, daß - wenn n und 1 jeweils für die Ziffer 0 stehen - k und r nicht gleichzeitig jeweils die Ziffer 1 bedeuten und daß gewünschtenfalls der Rest der Säuregruppen als Ester oder Amid vorliegt, sowie deren Salze mit anorganischen und/oder organischen Basen, Aminosäuren oder Aminosäureamide.
dadurch gekennzeichnet, daß man in an sich bekannter Weise Verbindungen der allgemeinen Formeln II und III^{.} 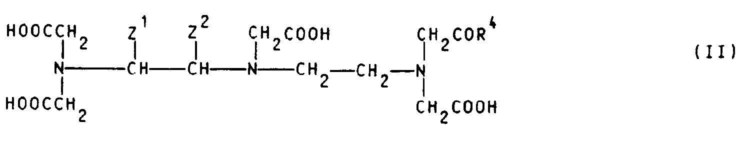 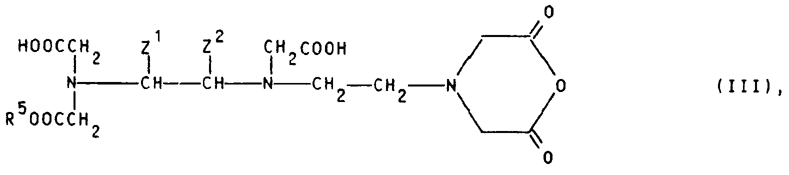 worin
Z¹ und Z² die oben angegebenen Bedeutungen haben,
R⁴ für den Rest einer aktivierten Carbonylgruppe oder für OR⁵ steht und
R⁵ ein Wasserstoffatom oder einen C₁-C₆-Alkylrest darstellt, mit Aminosäuren der allgemeinen Formel IV 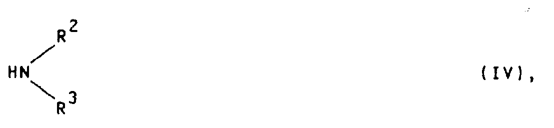
worin
R² und R³ die oben genannten Bedeutungen haben,
umsetzt, die so erhaltenen Komplexbildnersäuren der allgemeinen Formel I mit X in der Bedeutung eines Wasserstoffatoms mit mindestens einem Metalloxid oder Metallsalz eines Elements der Ordnungszahlen 21-29, 31, 32, 37-40, 42-44, 49 oder 57-83 umsetzt und anschließend, falls gewünscht. noch vorhandene acide Wasserstoffatome durch Kationen anorganischer und/oder organischer Basen, Aminosäuren oder Aminosäureamide substituiert bzw. die entsprechenden Säuregruppen ganz oder teilweise in Ester oder Amide überführt.

12. Verfahren zur Herstellung der pharmazeutischen Mittel gemäß Anspruch 8, dadurch gekennzeichnet, daß man die in Wasser, physiologischer Salzlösung oder Proteinlösung gelöste oder suspendierte Komplexverbindung, gegebenenfalls mit den in der Galenik üblichen Zusätzen, in eine für die enterale oder parenterale Applikation geeignete Form bringt.

## Claims

1. Compounds of the general formula I 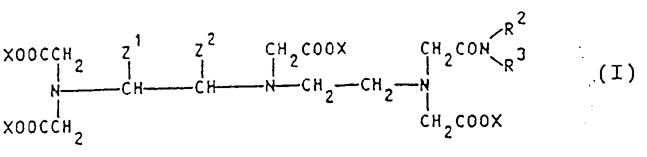 wherein
Z¹ and Z² each represents a hydrogen atom or the radical
-(CH₂)ₘ-(C₆H₄)_{q}-(O)ₖ-(CH₂)ₙ-(C₆H₄)ₗ-(O)ᵣ-R
wherein
m and n represent the numbers 0 to 20,
k, l, q and r represent the numbers 0 and 1, and
R represents a hydrogen atom, an optionally OR¹-substituted C₁-C₆-alkyl radical or a CH₂COOR¹ group wherein R¹ represents a hydrogen atom, a C₁-C₆-alkyl radical or a benzyl group,
R² represents a saturated, unsaturated, straight-chain or branched-chain or cyclic alkyl group having up to 20 carbon atoms, an aryl group or an aralkyl group, each substituted by a carboxy or sulfo group optionally esterified by a C₁-C₆-alkyl or benzyl radical,
R³ represents a hydrogen atom or a saturated, unsaturated, straight-chain or branched-chain or cyclic alkyl group having up to 20 carbon atoms, an aryl group or an aralkyl group, each optionally substituted by a carboxy or sulfo group optionally esterified by a C₁-C₆-alkyl or benzyl radical,
X represents a hydrogen atom and/or an equivalent of a metal ion of an element of atomic number 21-29, 31, 32, 37-40, 42-44, 49 or 57-83, with the proviso that at least one of the substituents Z¹ and Z² represents a hydrogen atom, that - when n and 1 each represents the number 0 - k and r do not at the same time each represent the number 1, and that, if desired, the radical of the acid groups is in the form of an ester or an amide, and salts thereof with inorganic and/or organic bases, amino acids or amino acid amides.

2. Compounds according to claim 1, characterised in that at least two of the substituents X are metal ion equivalents of at least one element of atomic number 21-29, 42, 44 or 57-83, or of at least one radionuclide of an element of atomic number 27, 29, 31, 32, 37-39, 43, 49, 62, 64, 70 or 77.

3. Compounds according to claim 1, characterised in that Z¹ and Z² each represents a hydrogen atom.

4. Compounds according to claim 1, characterised in that Z¹ represents a hydrogen atom and Z² represents the radical -(CH₂)ₘ-(C₆H₄)_{q}-(O)ₖ-(CH₂)ₙ-(C₆H₄)ₗ-(O)ᵣ-R.

5. Compounds according to claim 1, characterised in that Z² represents a hydrogen atom and Z₁ represents the radical -(CH₂)ₘ-(C₆H₄)_{q}-(O)ₖ-(CH₂)ₙ-(C₆H₄)ₗ-(O)ᵣ-R.

6. Compounds according to claim 1, characterised in that Z¹ or Z² represents the radicals -CH₂-C₆H₄-OH, -CH₂-C₆H₄-OCH₃, -CH₂-C₆H₅, -CH₂-C₆H₄-O-CH₂-C₆H₄-OCH₃, CH₂-O-CH₂-C₆H₅, -CH₂-C₆H₄-O-CH₂-COOH, -CH₂-C₆H₄-OC₂H₅, -CH₂-C₆H₄-OC₄H₉, -CH₂-C₆H₄-O-CH₂-C₆H₅.

7. Compounds according to claim 1, characterised in that R² or R³ represents a methyl, ethyl, propyl, isopropyl, butyl, pentyl, hexyl, phenyl, benzyl, 4-carboxy-phenylene, 1-cyclohexyl-1-carboxylic acid, 1-cyclopentyl-1-carboxylic acid, 2-carboxy-phenylene, 11-carboxy-1-undecyl, 10-carboxy-1-decyl, 7-carboxy-1-heptyl, 5-carboxy-1-hexyl, 5-carboxy-1-pentyl, 4-carboxy-benzyl, 4-carboxy-1-cyclohexylmethyl, 4-carboxymethyl-phenylene, 4-(1,1-dimethylcarboxymethylene)-phenylene, 4-sulfophenylene, 10-sulfo-1-decyl, 8-sulfo-1-octyl, 17-carboxy-7-heptadecyl, 7-carboxy-2-heptyl, 1-carboxy-4-phenyl-2-propyl, 2-carboxyethyl, 4-carboxy-4-but-1-enyl radical.

8. Pharmaceutical agents containing at least one physiologically tolerable compound according to claim 1, optionally together with the additives customary in galenical pharmacy.

9. The use of at least one physiologically tolerable compound according to claim 1 for the production of agents for NMR, X-ray, radiodiagnostic, radio or radiation therapy.

10. The use of at least one physiologically tolerable compound according to claim 1 for the production of agents for NMR diagnosis of the gastro-intestinal tract, the renal system and the hepatobiliary system.

11. Process for the production of compounds of the general formula I 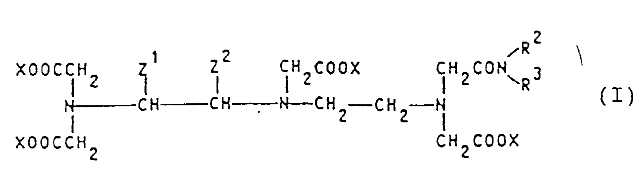 wherein
Z¹ and Z² each represents a hydrogen atom or the radical
-(CH₂)ₘ-(C₆H₄)_{q}-(O)ₖ-(CH₂)ₙ-(C₆H₄)ₗ-(O)ᵣ-R
wherein
m and n represent the numbers 0 to 20,
k, l, q and r represent the numbers 0 and 1, and
R represents a hydrogen atom, an optionally OR¹-substituted C₁-C₆-alkyl radical or a CH₂COOR¹ group wherein R¹ represents a hydrogen atom, a C₁-C₆-alkyl radical or a benzyl group,
R² represents a saturated, unsaturated, straight-chain or branched-chain or cyclic alkyl group having up to 20 carbon atoms, an aryl group or an aralkyl group, each substituted by a carboxy or sulfo group optionally esterified by a C₁-C₆-alkyl or benzyl radical,
R³ represents a hydrogen atom or a saturated, unsaturated, straight-chain or branched-chain or cyclic alkyl group having up to 20 carbon atoms, an aryl group or an aralkyl group, each optionally substituted by a carboxy or sulfo group optionally esterified by a C₁-C₆-alkyl or benzyl radical,
X represents a hydrogen atom and/or an equivalent of a metal ion of an element of atomic number 21-29, 31, 32, 37-40, 42-44, 49 or 57-83, with the proviso that at least one of the substituents Z¹ and Z² represents a hydrogen atom, that - when n and l each represents the number 0 - k and r do not at the same time each represent the number 1, and that, if desired, the radical of the acid groups is in the form of an ester or an amide, and salts thereof with inorganic and/or organic bases, amino acids or amino acid amides,
characterised in that, in a manner known per se, compounds of the general formulae II and III 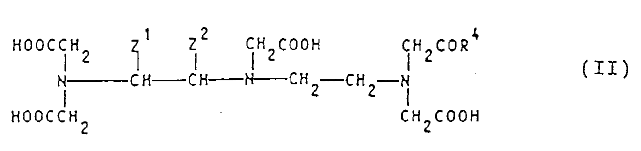 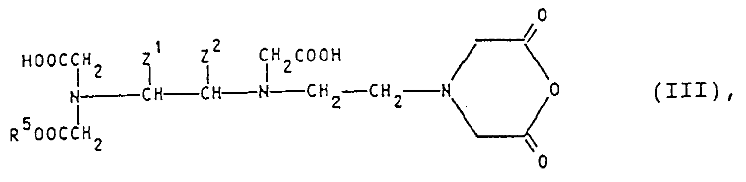 wherein
Z¹ and Z² have the meanings given above,
R⁴ represents the radical of an activated carbonyl group or represents OR⁵, and
R⁵ represents a hydrogen atom or a C₁-C₆-alkyl radical,
are reacted with amino acids of the general formula IV 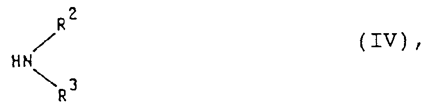 wherein
R² and R³ have the meanings given above,
the resulting complex-forming acids of the general formula I wherein X represents a hydrogen atom are reacted with at least one metal oxide or metal salt of an element of atomic number 21-29, 31, 32, 37-40, 42-44, 49 or 57-83 and then, if desired, any acid hydrogen atoms still present are replaced by cations of inorganic and/or organic bases, amino acids or amino acid amides, or the corresponding acid groups are completely or partially converted into esters or amides.

12. Process for the production of pharmaceutical agents according to claim 8, characterised in that the complex compound dissolved or suspended in water, physiological saline solution or protein solution is brought into a form suitable for enteral or parenteral administration, optionally together with the additives customary in galenical pharmacy.

## Revendications

1. Composés de formule générale I ci-dessous : 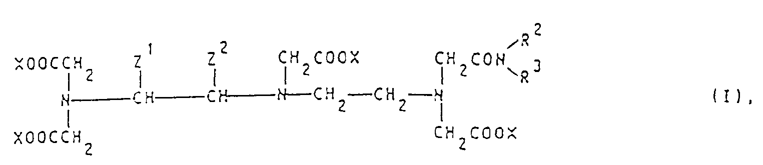 (dans laquelle
Z¹ et Z² représentent chacun un atome d'hydrogène ou un radical
-(CH₂)ₘ-(C₆H₄)_{q}-(O)ₖ-(CH₂)ₙ-(C₆H₄)ₗ-(O)ᵣ-R,
m et n étant des nombres de 0 à 20,
k, l, q et r le nombre 0 ou 1 et
R un atome d'hydrogène, un alkyle en C₁-C₆ avec éventuellement un substituant -OR¹ ou un groupe - CH₂COOR¹, R¹ désignant un atome d'hydrogène, un alkyle en C₁-C₆ ou le groupe benzyle,
R² représente un aryle, un aralkyle ou un alkyle pouvant avoir jusqu'à 20 atomes de carbone, saturé ou insaturé, linéaire, ramifié ou cyclique, substitué par un groupe carboxylique ou sulfonique éventuellement estérifié par un alkyle en C₁-C₆ ou un benzyle,
R³ un atome d'hydrogène ou un aryle, un aralkyle ou un alkyle pouvant avoir jusqu'à 20 atomes de carbone, saturé ou insaturé, linéaire, ramifié ou cyclique, avec éventuellement comme substituant un groupe carboxylique ou sulfonique le cas échéant estérifié par un alkyle en C₁-C₆ ou un benzyle, et
X un atome d'hydrogène et/ou un équivalent d'ion de métal d'un élément des numéros atomiques 21-29, 31, 32, 37-40, 42-44, 49 et 57-83, avec les conditions que l'un au moins des substituants Z¹ et Z² soit un atome d'hydrogène, que si n et l sont chacun le nombre 0 k et r ne soient pas à la fois chacun le nombre 1, et que si l'on veut le radical des groupes d'acides soit en un ester ou un amide), ainsi que leurs sels formés avec des bases minérales et/ou organiques, aminoacides ou amides d'aminoacides.

2. Composés selon la revendication 1, caractérisés en ce que au moins deux des substituants X sont des équivalents d'ions de métaux d'un ou plusieurs des éléments de numéros atomiques 21-29, 42, 44 et 57-83, ou bien au moins un radionucléide d'un élément des numéros atomiques 27, 29, 31, 32, 37-39, 43, 49, 62, 64, 70 et 77.

3. Composés selon la revendication 1, caractérisés en ce que Z¹ et Z² sont chacun l'hydrogène.

4. Composés selon la revendication 1, caractérisés en ce que Z¹ est un atome d'hydrogène et Z² un radical - (CH₂)ₘ-(C₆H₄)_{q}-(O)ₖ-(CH₂)ₙ-(C₆H₄)ₗ-(O)ᵣ-R.

5. Composés selon la revendication 1, caractérisés en ce que Z² est un atome d'hydrogène et Z¹ un radical - (CH₂)ₘ-(C₆H₄)_{q}-(O)ₖ-(CH₂)ₙ-(C₆H₄)ₗ-(O)ᵣ-R.

6. Composés selon la revendication 1, caractérisés en ce que Z¹ et/ou Z² sont les radicaux
-CH₂-C₆H₄-OH, -CH₂-C₆H₄-OCH₃, -CH₂-C₆H₅, -CH₂-C₆H₄-O-CH₂-C₆H₄-OCH₃, -CH₂-O-CH₂-C₆H₅, -CH₂-C₆H₄-O-CH₂-COOH, -CH₂-C₆H₄-OC₂H₅, -CH₂-C₆H₄-OC₄H₉ ou -CH₂-C₆H₄-O-CH₂-C₆H₅.

7. Composés selon la revendication 1, caractérisés en ce que R² et/ou R³ sont un groupe méthyle, éthyle, propyle, isopropyle, butyle, pentyle, hexyle, phényle, benzyle, 4-carboxy-phénylène, 1-cyclohexyle-1-carboxylique, 1-cyclopentyl-1-carboxylique, 2-carboxy-phénylène, 11-carboxy-1-undécyle, 10-carboxy-1-décyle, 7-carboxy-1-heptyle, 6-carboxy-1-hexyle, 5-carboxy-1-pentyle, 4-carboxy-benzyle, 4-carboxy-1-cyclohexylméthyle, 4-carboxyméthylphénylène, 4-(1,1-diméthylcarboxyméthylène)-phénylène, 4- sulfo-phénylène, 10-sulfo-1-décyle, 8-sulfo-1-octyle, 17-carboxy-7-heptadécyle, 7-carboxy-2-heptyle, 1-carboxy-4-phényl-2-propyle, 2-carboxyéthyle ou 4-carboxy-4-but-1-ényle.

8. Produits pharmaceutiques contenant un ou plusieurs composés de 12 revendication 1 physiologiquement compatibles et tolérés, éventuellement avec les additifs courants en galénique.

9. L'emploi de composés de la revendication 1 physiologiquement compatibles pour en fabriquer des produits destinés à des diagnostics par RMN, rayons X ou radio-diagnostics, et à la radiothérapie ou thérapie par rayonnements.

10. Emploi des composés de la revendication 1 physiologiquement compatibles pour en fabriquer des agents destinés au diagnostic par RMN de l'appareil gastro-intestinal, du système rénal et du système hépatobiliaire.

11. Procédé de préparation des composés de formule générale I : 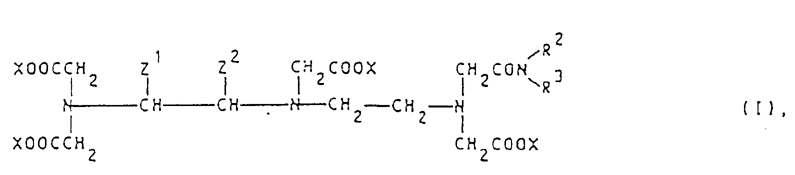 (dans laquelle
Z¹ et Z² représentent chacun un atome d'hydrogène ou un radical
- (CH₂)ₘ- (C₆H₄)_{q}- (O)ₖ- (CH₂)ₙ- (C₆H₄)ₗ- (O)ᵣ-R,
m et n étant des nombres de 0 à 20,
k, l, q et r le nombre 0 ou 1 et
R un atome d'hydrogène, un alkyle en C₁-C₆ avec éventuellement un substituant -OR¹ ou un groupe - CH₂COOR¹, R¹ désignant un atome d'hydrogène, un alkyle en C₁-C₆ ou le groupe benzyle,
R² représente un aryle, un aralkyle ou un alkyle pouvant avoir jusqu'à 20 atomes de carbone, saturé ou insaturé, linéaire, ramifié ou cyclique, substitué par un groupe carboxylique ou sulfonique éventuellement estérifié par un alkyle en C₁-C₆ ou un benzyle,
R³ un atome d'hydrogène ou un aryle, un aralkyle ou un alkyle pouvant avoir jusqu'à 20 atomes de carbone, saturé ou insaturé, linéaire, ramifié ou cyclique, avec éventuellement comme substituant un groupe carboxylique ou sulfonique le cas échéant estérifié par un alkyle en C₁-C₆ ou un benzyle, et
X un atome d'hydrogène et/ou un équivalent d'ion de métal d'un élément des numéros atomiques 21-29, 31, 32, 37-40, 42-44, 49 et 57-83, avec les conditions que l'un au moins des substituants Z¹ et Z² soit un atome d'hydrogène, que si n et l sont chacun le nombre 0 k et r ne soient pas à la fois chacun le nombre 1, et que si l'on veut le radical des groupes d'acides soit en un ester ou un amide), ainsi que de leurs sels avec des bases minérales et/ou organiques, aminoacides ou amides d'aminoacides,
procédé caractérisé en ce que l'on fait réagir de manière connue des composés de formules II et III : 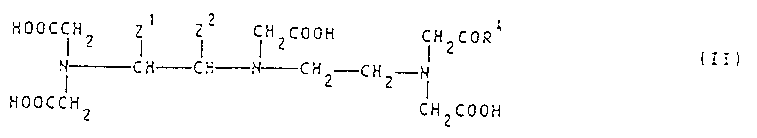 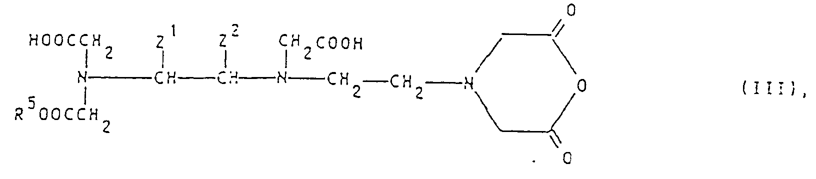 dans lesquelles :
Z¹ et Z² ont les significations précédemment données,
R⁴ est le radical d'un groupe carbonylique activé ou un groupe OR⁵, et
R⁵ un atome d'hydrogène ou un alkyle en C₁-C₆, avec des aminoacides de formule IV : 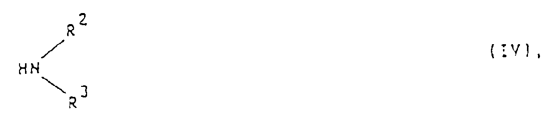
R² et R³ ayant les significations précédemment données,
puis on fait réagir les acides formateurs de complexes ainsi obtenus de formule I où X est un atome d'hydrogène avec un ou plusieurs oxydes ou sels de métaux d'éléments des numéros atomiques 21-29, 31, 32, 37-40, 42-44, 49 ou 57-83, et ensuite, si l'on veut, on remplace des atomes d'hydrogène acides encore présents par des cations de bases minérales et/ou organiques, d'aminoacides ou d'amides d'aminoacides, et/ou on transforme les groupes acides correspondants, en totalité ou en partie, en esters ou amides.

12. Procédé de préparation des produits pharmaceutiques de la revendication 8, procédé caractérisé en ce que l'on met sous une forme appropriée à ces administrations entérales ou parentérales le composé complexe en solution ou en suspension dans de l'eau, dans du sérum physiologique ou dans une solution de protéine, éventuellement avec les additifs courants en pharmacie galénique.
